# DEMANDE DE BREVET EUROPEEN

(11) **EP 2 353 582 A1**
(43) Date de publication de la demande: **10.08.2011**
(21) Numéro de dépôt: 10193776.1
(22) Date de dépôt: 06.12.2010
(51) Int. Cl.: A61K 8/41, A61K 8/87, A61Q 1/00, A61Q 1/14, A61K 8/04, A61K 8/19, A61Q 1/10

(54) **Composition de maquillage des cils et des sourcils**

(30) Priorité: 18.12.2009 FR 0959173; 18.12.2009 FR 0959178; 18.12.2009 FR 0959177; 02.02.2010 US 300460 P; 02.02.2010 US 300461 P; 02.02.2010 US 300462 P
(71) Demandeur: L'Oréal, 75008 Paris (FR)
(72) Inventeur: Kergosien, Guillaume, 92370, Chaville (FR); Jager Lezer, Nathalie, 91370, Verrieres-le-Buisson (FR); Portois, Emmanuelle, 94600, Choisy le Roi (FR)
(74) Mandataire: Goudet, Sylvain

(57) **Abrégé**

La présente invention concerne une composition de maquillage sur des fibres kératiniques, telles que des cils ou des sourcils, aux propriétés démaquillantes améliorées, comprenant dans une phase aqueuse continue :
- une dispersion aqueuse de polyuréthane particulier présent en une quantité en matière sèche supérieure ou égale à 5% en poids par rapport au poids total de ladite composition, et
- ladite composition comprenant un système émulsionnant comprenant moins de 1% en poids de triéthanolamine, et comprenant une matière colorante choisie parmi les matières pulvérulentes.

## Description

La présente invention concerne une composition de maquillage des matières kératiniques, adaptés à former des films de maquillage sur les fibres kératiniques, telles que les cils ou sourcils, et aisément démaquillables.

Le film de maquillage peut être formé partir d'une composition se présentant sous la forme d'un mascara, ou d'un produit pour les sourcils. Plus préférentiellement, l'invention porte sur le démaquillage et/ou le nettoyage d'un mascara. Par « mascara », on entend une composition destinée à être appliquée sur les cils : il peut s'agir d'une composition de maquillage des cils, une base de maquillage des cils (aussi appelée base coat), une composition à appliquer sur un mascara, dite encore top-coat, ou bien encore une composition de traitement cosmétique des cils. Le mascara est plus particulièrement destiné aux cils d'êtres humains, mais également aux faux-cils.

Les compositions de maquillage des cils ou mascaras peuvent être constituées d'au moins une cire ou d'un mélange de cires dispersé dans une phase liquide aqueuse ou solvant organique. Ils présentent en général une texture pâteuse et sont conditionnés dans un récipient. Ce récipient définit un réservoir muni d'un essoreur et obturé par un applicateur. Cet applicateur peut se présenter notamment sous forme d'une brosse ou d'un peigne configuré pour prélever du produit contenu dans le réservoir. Un tel applicateur peut alors être débarrassé du surplus de produit lors de son passage à travers l'essoreur puis être mis en contact des fibres kératiniques à maquiller, telles que les cils ou les sourcils.

Toutefois, le film de maquillage obtenu après l'application de ces compositions n'est généralement pas suffisamment résistant à l'eau, lors de baignades ou de douches par exemple, aux larmes ou à la sueur ou encore au sébum. Le mascara a alors tendance à couler ― apparitions d'auréoles sous les yeux - ou à s'effriter dans le temps : des grains se déposent et des traces inesthétiques apparaissent autour des yeux.

Afin de résoudre le problème susmentionné, des dispersions de polyuréthane ont été récemment incorporées dans les produits cosmétiques offrant plusieurs avantages par rapport aux technologies classiques telles que les acryliques et les copolymères d'acrylamide, la polyvinylpyrrolidone, et les copolymères de PVP/VA. Ces avantages comprennent la compatibilité avec l'eau, la résistance à l'eau et une excellente aptitude à former des films. Toutefois, de telles dispersions peuvent souffrir d'un manque d'adhérence sur la surface enduite ce qui se traduit par un effritement de la composition. Ceci crée un problème esthétique important pour les consommateurs. De tels exemples de dispersions de polyuréthane sont en particulier fournis dans les demandes EP 1 970 391 et EP 2 105 126.

Le brevet US 6 106 813 divulgue également des polyester-polyuréthanes qui conviennent dans des applications cosmétiques. Il divulgue une nouvelle famille de polyester-polyuréthanes qui présente non seulement de bonnes propriétés filmogènes, mais qui confère également une grande rigidité et une excellente résistance à l'élimination par l'eau et les détergents.

Ce type de composition permet ainsi en général d'obtenir un film ayant de bonnes propriétés de non transfert, de bonnes propriétés de tenue dans le temps, en particulier à l'eau et aux frottements, et formant un dépôt confortable sur la peau, les lèvres, les cils ou les ongles. Ainsi, cette nouvelle voie de formulation permet d'améliorer les performances de produits de maquillage, de soin ou de traitement des matières kératiniques.

En revanche, les films formés par de telles dispersions sont plus difficiles à éliminer que des produits classiques.

Un but de la présente invention réside donc dans la mise au point d'une composition de maquillage qui, tout en conservant une bonne tenue sur les cils ou sourcils, voire améliorant cette tenue, présente par ailleurs une bonne aptitude au démaquillage.

Par conséquent, il subsiste le besoin de fournir des compositions formant des films de maquillage de bonne tenue tout en étant facilement éliminable.

Pour ce faire, la présente invention a pour objet une composition de maquillage des fibres kératiniques, telle que les cils ou sourcils, aux propriétés démaquillantes améliorées, comprenant dans une phase aqueuse continue :
- une dispersion aqueuse de polyuréthane, le polyuréthane dispersé comprenant les produits de réaction de :
   A) un prépolymère selon la formule : 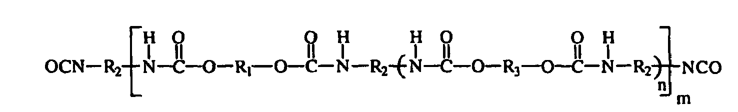 où
      R₁ représente un radical hydrocarboné issu d'un polyesterpolyol, et notamment d'un polyesterdiol,
      R₂ représente un radical hydrocarboné issu d'un polyisocyanate aliphatique ou cycloaliphatique,
      R₃ représente un radical hydrocarboné issu d'un diol, éventuellement de bas poids moléculaire, éventuellement substitué par des groupes ioniques,
      n vaut 0 à 5, et
      m est > 1 ;
   B) au moins un agent d'allongement de chaîne selon la formule :
      H₂N—R₄—NH₂
      où
      R₄ représente un radical alkylène ou oxyde d'alkylène non substitué par des groupes ioniques ou potentiellement ioniques ; et
   C) au moins un agent d'allongement de chaîne selon la formule :
      H₂N-R₅-NH₂
      où
      R₅ représente un radical alkylène substitué par des groupes ioniques ou
      potentiellement ioniques ;
- ledit polyuréthane étant présent en une quantité en matière sèche supérieure ou égale à 5% en poids par rapport au poids total de ladite composition, et
- ladite composition comprenant un système émulsionnant comprenant moins de 1% en poids de triéthanolamine, et comprenant au moins une matière colorante choisie parmi les matières pulvérulentes.

La présente invention concerne également un procédé de démaquillage et/ou de nettoyage des fibres kératiniques, telle que les cils ou sourcils, d'une composition de maquillage telle que précédemment définie appliquée sur les cils, ledit procédé comprenant une étape consistant à appliquer sur le film de maquillage une composition démaquillante et/ou nettoyante.

Ledit procédé peut éventuellement comprendre une étape consistant à exercer une traction sur le film de maquillage de la base de la fibre kératinique vers l'extrémité libre de ladite fibre en vue d'éliminer au moins partiellement ledit film. Cette étape peut se faire manuellement et notamment à l'aide d'un support tissé ou non tissé, éventuellement laminé, tel qu'un disque demakeup®. Ce support peut être anhydre. En variante, ce support peut être pré-imprégné de la composition démaquillante et/ou nettoyante. Cette composition démaquillante et/ou nettoyante peut être de l'eau ou un solvant hydrosoluble.

Les compositions démaquillante et/ou nettoyante peuvent comprendre essentiellement de l'eau et/ou un ou plusieurs solvant(s) hydrosoluble(s), par exemple présent(e)(s) à une quantité allant de 50 à 100% en poids par rapport au poids total de la composition. Ces compositions peuvent également comprendre une phase huileuse.

De telles compositions peuvent se présenter sous la forme de lotions, éventuellement biphasiques, de laits, de crèmes ou encore de gels.

Les inventeurs ont découvert que ces films de maquillage particuliers peuvent être aisément éliminés par un geste conventionnel de traction permettant un démaquillage par gaine, sans effritement substantiel des films de maquillage. Un tel démaquillage permet ainsi d'éviter de disperser de la composition de maquillage lors de son retrait des fibres kératiniques.

Par gaines, on entend la formation de manchons de longueur supérieure ou égale à 1 mm, mieux d'au moins 2 mm.

Afin d'évaluer le résultat démaquillant et/ou de nettoyant de la composition selon la présente demande, on effectue un test in vitro selon le protocole suivant :
- On applique la composition sur 3 éprouvettes de cheveux caucasiens raides 30 noeuds (60 cils longs de 1 cm) longueur de la frange 2 cm en faisant 3 x 10 passages espacés de 2 minutes avec reprise de produit entre chaque série de 10.
- Chaque éprouvette est ensuite séchée à température ambiante pendant un temps de séchage d'une heure.
- Les 3 éprouvettes maquillées sont immergées dans un récipient contenant de l'eau à 20°C pendant un temps donné (1 heure, 24 heures ou 1 semaine). On effectue ensuite 5 allers-retours des 3 éprouvettes sur une chiffonnette carrée type Wypall L40 de chez Kimberly-clark.
- On dépose alors les 3 éprouvettes sur un support coton respectif du type disque demakup® puis on plie chaque coton en 2 autour de leur éprouvette respective.
- On applique une légère pression sur chaque coton et on tire lesdits cotons relativement aux éprouvettes.
- On observe alors visuellement le dépôt sur chaque coton.

La présente invention concerne également un ensemble ou kit de démaquillage des fibres kératiniques, telles que des cils ou des sourcils, comprenant :
- au moins une composition de maquillage ci-dessus décrite, et
- au moins une composition démaquillante et/ou nettoyante de cette composition de maquillage.

Les dispersions de polyuréthane de la présente invention conviennent particulièrement pour une utilisation dans les produits de maquillage des matières kératiniques et en particulier des fibres kératiniques, en particulier des cils ou sourcils.

La présente invention a pour objet selon un deuxième objet éventuellement indépendant du système émulsionnant mis en oeuvre, une composition de maquillage et/ou de soin des fibres kératiniques, telles que les cils ou sourcils, comprenant dans une phase aqueuse continue :
A) un prépolymère selon la formule : 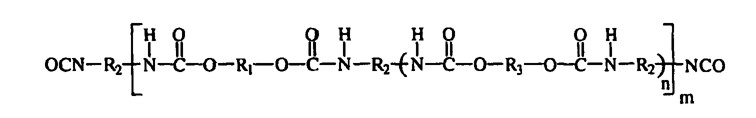 où
   R₁ représente un radical hydrocarboné divalent ou un composé à fonctionnalité dihydroxyle, avantageusement un radical issu d'un polyesterpolyol et notamment un polyesterdiol,
   R₂ représente un radical hydrocarboné issu d'un polyisocyanate aliphatique ou cycloaliphatique,
   R₃ représente un radical hydrocarboné issu d'un diol, éventuellement de bas poids moléculaire, éventuellement substitué par des groupes ioniques,
   n vaut 0 à 5, et
   m est > 1 ;
   B) au moins un agent d'allongement de chaîne selon la formule :
      H₂N—R₄—NH₂
      où
      R₄ représente un radical alkylène ou oxyde d'alkylène non substitué par des groupes ioniques ou potentiellement ioniques ; et
   C) au moins un agent d'allongement de chaîne selon la formule :
      H₂N-R₅-NH₂
      où
      R₅ représente un radical alkylène substitué par des groupes ioniques ou potentiellement ioniques ; et
- au moins un épaississant hydrophile choisi parmi les copolymères issus de la polymérisation :
   (i) d'au moins un monomère de formule (1) suivante : 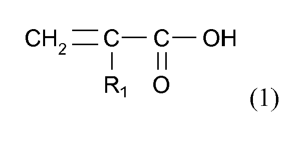 dans laquelle, R₁ désigne H ou CH₃ ou C₂H₅, c'est-à-dire des monomères acide acrylique, acide méthacrylique ou acide éthacrylique, et
   (ii) d'au moins un monomère de type ester d'alkyl (C₁₀-C₃₀) d'acide carboxylique insaturé correspondant au monomère de formule (2) suivante : 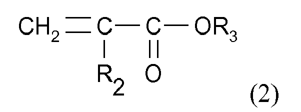 dans laquelle, R₂ désigne H ou CH₃ ou C₂H₅ et de préférence H ou CH₃, R₃ désignant un radical alkyle en C₁₀-C₃₀, et de préférence en C₁₂-C₂₂.

D'une manière inattendue, les inventeurs ont découvert que l'utilisation de polyuréthanes particuliers formulés dans une phase aqueuse continue en présence d'un épaississant hydrophile tel que précédemment défini permet d'obtenir une composition présentant une excellente tenue à l'eau et/ou au sébum, et le cas échéant une très bonne résistance aux frottements.

Avantageusement, les esters d'alkyles (C₁₀-C₃₀) d'acides carboxyliques insaturés sont choisis de préférence parmi l'acrylate de lauryle, l'acrylate de stéaryle, l'acrylate de décyle, l'acrylate d'isodécyle, l'acrylate de dodécyle, et les méthacrylates correspondants, tels que le méthacrylate de lauryle, le méthacrylate de stéaryle, le méthacrylate de décyle, le méthacrylate d'isodécyle, et le méthacrylate de dodécyle et leurs mélanges.

Selon un mode de réalisation préféré, ces polymères épaississants sont réticulés.

Selon un mode de réalisation particulier, ledit épaississant hydrophile est choisi parmi des polymères issus de la polymérisation d'un mélange de monomères comprenant :
(i) essentiellement de l'acide acrylique,
(ii) un ester de formule (2) décrite ci-dessus et dans laquelle R₂ désigne H ou CH₃, R₃ désignant un radical alkyle ayant de 12 à 22 atomes de carbone, et
(iii) un agent réticulant, tel qu'un un monomère insaturé polyéthylénique copolymérisable, comme le phtalate de diallyle, le (méth)acrylate d'allyl, le divinylbenzène, le diméthacrylate de (poly)éthylèneglycol, et le méthylène-bis-acrylamide.

Ladite composition comprend avantageusement :
(i) de 95 à 60% en poids d'acide acrylique,
(ii) de 4 à 40% en poids d'acrylate d'alkyles en C₁₀-C₃₀, et
(iii) de 0 à 6% en poids de monomère polymérisable réticulant.

Ladite composition comprend avantageusement :
(i) de 98 à 96% en poids d'acide acrylique,
(ii) de 1 à 4% en poids d'acrylate d'alkyles en C_{IO}-C₃₀, et
(iii) de 0,1 à 0.6% en poids de monomère polymérisable réticulant.

Différents tests pour évaluer cette tenue par rapport à l'art antérieur ont été réalisés pour montrer les effets avantageux apportés par la solution technique proposée. Les résultats des tests pratiqués relativement à cet objet de l'invention seront présentés ultérieurement dans cette description.

La présente invention a encore pour objet selon un troisième objet éventuellement indépendant du système émulsionnant mis en oeuvre, et éventuellement indépendant de la présence ou non d'épaississant hydrophile, une composition de maquillage et/ou de soin des fibres kératiniques comprenant :
- une dispersion aqueuse de polyuréthane, le polyuréthane dispersé comprenant les produits de réaction de :
   A) un prépolymère selon la formule : 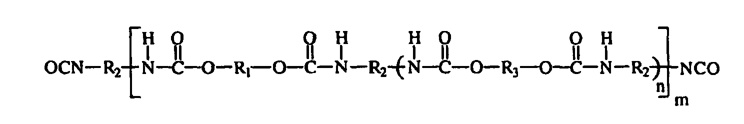 où
      R₁ représente un radical hydrocarboné divalent ou un composé à fonctionnalité dihydroxyle, avantageusement un radical issu d'un polyesterdiol,
      R₂ représente un radical hydrocarboné issu d'un polyisocyanate aliphatique ou cycloaliphatique,
      R₃ représente un radical hydrocarboné issu d'un diol, éventuellement de bas poids moléculaire, éventuellement substitué par des groupes ioniques,
      n vaut 0 à 5, et
      m est > 1 ;
   B) au moins un agent d'allongement de chaîne selon la formule :
      H₂N—R₄—NH₂
      où
      R₄ représente un radical alkylène ou oxyde d'alkylène non substitué par des groupes ioniques ou potentiellement ioniques ; et
   C) au moins un agent d'allongement de chaîne selon la formule :
      H₂N—R₅—NH₂
      où
      R₅ représente un radical alkylène substitué par des groupes ioniques ou potentiellement ioniques ; et
- au moins un agent plastifiant lipophile présent à une quantité en poids supérieure ou égale à 2% par rapport au poids total de la composition.

D'une manière inattendue, les inventeurs ont découvert que l'utilisation de polyuréthanes particuliers formulés dans une phase aqueuse continue en présence d'un plastifiant présent en une quantité en poids strictement supérieure à 2% par rapport au poids total de la composition permet d'obtenir une composition présentant une très bonne tenue à l'eau et/ou au sébum, et le cas échéant une très bonne résistance aux frottements.

Avantageusement, cet agent plastifiant est choisi parmi :
- les (poly)esters issus de la ou des réaction(s) d'au moins un acide carboxylique avec au moins un (poly)ol,
- les éthers de glycol,
- le N-éthyl-o,p-toluènesulfonamide,
- les carbonates,
- les cétones,
et leurs mélanges.

Selon un mode de réalisation préféré, ledit agent plastifiant est choisi parmi les esters d'acide citrique, tels que le citrate de triéthyle, le citrate de tributyle, l'acétylcitrate de triéthyle, l'acétylcitrate de tributyle, l'acétylcitrate de triéthyl-2 hexyle, et leurs mélanges.

Là encore, différents tests pour évaluer cette tenue par rapport à l'art antérieur ont été réalisés pour montrer les effets avantageux apportés par la solution technique proposée, qui seront dévoilés ultérieurement dans cette description.

Sauf mention expresse, la description qui suit est adaptée aux trois objets indépendants précédemment énoncés.

### POLYURETHANES

Le polyuréthane peut être présent en une quantité en matière sèche supérieure ou égale à 5% en poids par rapport au poids total de la composition, mieux 6.5% en poids voire 8% en poids ou plus. Le polyuréthane peut par exemple être présent à une quantité en matière sèche allant de 5% et 30% en poids par rapport au poids total de ladite composition, mieux allant de 6 à 25%, mieux de 7 à 15%.

Les compositions selon l'invention comprennent des dispersions aqueuses de particules de polyuréthanes particuliers. De telles compositions peuvent être utilisées dans des applications cosmétiques, de maquillage ou de soin des cils ou sourcils.

Le polyuréthane est un produit de la réaction de :
A) un prépolymère selon la formule : 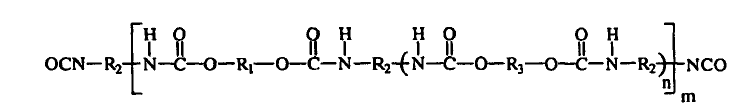 où
   R₁ représente un radical hydrocarboné issu d'un polyesterpolyol, et notamment d'un polyesterdiol,
   R₂ représente un radical hydrocarboné issu d'un polyisocyanate aliphatique ou cycloaliphatique,
   R₃ représente un radical hydrocarboné issu d'un diol, éventuellement de bas poids moléculaire, éventuellement substitué par des groupes ioniques,
   n vaut 0 à 5, et
   m est > 1 ;
B) au moins un agent d'allongement de chaîne selon la formule :
   H₂N—R₄—NH₂
   où
   R₄ représente un radical alkylène ou oxyde d'alkylène non substitué par des groupes ioniques ou potentiellement ioniques ; et
C) au moins un agent d'allongement de chaîne selon la formule :
   H₂N—R₅—NH₂
   où
   R₅ représente un radical alkylène substitué par des groupes ioniques ou potentiellement ioniques.

### Prépolymère A)

Les compositions selon l'invention comprennent des dispersions aqueuses de polyuréthanes particuliers. De telles compositions peuvent être utilisées dans des applications cosmétiques, de maquillage ou de soin des cils ou sourcils.

### Radical R1

Des composés appropriés pour fournir le radical polyhydroxyle, de préférence dihydroxyle, R₁ sont les polyesterpolyols, de préférence les polyesterdiols, et leurs mélanges. Ces composés sont ainsi avantageusement divalents, de préférence deux groupes hydroxy.

De tels composés peuvent présenter des poids moléculaires numériques moyens d'environ 700 à environ 16 000, et de préférence d'environ 750 à environ 5000.

Le(s) polyesterdiol(s) peuvent généralement être préparés à partir :
- d'acides dicarboxyliques ou polycarboxyliques aliphatiques, cycloaliphatiques ou aromatiques ou leurs anhydrides et
- d'alcools dihydriques tels que des diols choisis parmi les diols aliphatiques, alicycliques, aromatiques.

Les acides dicarboxyliques ou polycarboxyliques aliphatiques peuvent être choisis parmi : l'acide succinique, fumarique, glutarique, 2,2-diméthylglutarique, adipique, itaconique, pimélique, subérique, azélaïque, sébacique, maléique, malonique, 2,2-diméthylmalonique, nonanedicarboxylique, décanedicarboxylique, dodécanedioïque, 1,3-cyclohexanedicarboxylique, 1,4-cyclohexa-nedicarboxylique, 2,5-norborane dicarboxylique, diglycolique, thiodipropionique, 2,5-naphtalènedicarboxylique, 2,6-naphtalène-dicarboxylique, phtalique, téréphtalique, isophtalique, oxanique, o-phtalique, tétrahydrophtalique, hexahydrophtalique ou trimellitique.

Les d'anhydrides d'acides peuvent être en particulier choisi parmi l'anhydride de l'acide o-phtalique, trimellitique ou succinique ou un mélange de ceux-ci.

De préférence l'acide dicarboxylique préféré est l'acide adipique.

L'alcools dihydriques peut être choisi parmi l'éthanediol, l'éthylène glycol, le diéthylèneglycol, le triéthylène glycol, le triméthylèneglycol, le tétraéthylèneglycol, le 1,2-propanediol, le dipropylèneglycol, le tripropylèneglycol, le tétrapropylèneglycol, le 1,3-propanediol, le 1,4-butanediol, le 1,3-butanediol, le 2,3-butanediol, le 1,5-pentanediol, le 1,6-hexanediol, le 2,2-diméthyl-1,3-propanediol, le 1,4-dihydroxycyclohexane, le 1,4-diméthylolcyclohexane, le cyclohexane diméthanol, le 1,8-octanediol, le 1,10-décanediol, le 1,12-dodécanediol, le neopentylglycol ou leurs mélanges. Les composés dihydroxyliques cycloaliphatiques et/ou aromatiques conviennent bien entendu également comme alcool(s) dihydrique(s) pour la préparation de polyesterpolyol(s).

Les polyesterdiols peuvent aussi être choisis parmi les homopolymères ou copolymères de lactones, qui sont de préférence obtenus par des réactions d'addition de lactones ou des mélanges de lactones, tels que la butyrolactone, l'ε-caprolactone et/ou la méthyl-ε-caprolactone avec les molécules initiatrices polyfonctionnelles, de préférence difonctionnelles appropriées, telles que par exemple les alcools dihydriques cités ci-dessus. Les polymères correspondants d'ε-caprolactone sont préférés.

Le radical polyesterpolyol R₁, de préférence polyester diol, peut être obtenu avantageusement par polycondensation d'acides dicarboxyliques, tel que l'acide adipique, avec des polyols, notamment des diols, tels que l'hexanediol, le neopentylglycol, et leurs mélanges.

### Radical R2

Des polyisocyanates appropriés pour fournir le radical hydrocarboné R₂ comprennent les diisocyanates organiques présentant un poids moléculaire d'environ 112 à 1000, et de préférence d'environ 140 à 400.

Des diisocyanates préférés sont ceux représentés par la formule générale R₂(NCO)₂ indiquée ci-dessus, dans laquelle R₂ représente un groupe hydrocarboné aliphatique divalent comprenant 4 à 18 atomes de carbone, un groupe hydrocarboné cycloaliphatique divalent comprenant 5 à 15 atomes de carbone, un groupe hydrocarboné araliphatique divalent comprenant 7 à 15 atomes de carbone ou un groupe hydrocarboné aromatique divalent comprenant 6-15 atomes de carbone. Des exemples de diisocyanates organiques qui conviennent comprennent le tétraméthylènediisocyanate, le 1,6-hexaméthylènediisocyanate, le dodécaméthylènediisocyanate, le cyclohexane-1,3-diisocyanate et le cyclohexane-1,4-diisocyanate, le 1-isocyanato-3-isocyanatométhyl-3,5,5-triméthylcyclohexane (isophoronediisocyanate ou IPDI), le bis-(4-isocyanatocyclohexyl)-méthane, le 1,3-bis(isocyanatométhyl)-cyclohexane et le 1,4-bis(isocyanatométhyl)-cyclohexane, le bis-(4-isocyanato-3-méthyl-cyclohexyl)-méthane. Des mélanges de diisocyanates peuvent bien entendu être utilisés. Des diisocyanates préférés sont des diisocyanates aliphatiques et cycloaliphatiques. On préfère en particulier le 1,6-hexaméthylènediisocyanate, l'isophoronediisocyanate et le dicyclohexylméthane diisocyanate, et leurs mélanges.

### Radical R3

L'utilisation de diols, notamment de bas poids moléculaire, R₃ peut permettre une rigidification de la chaîne polymèrique et est optionnelle. L'expression "diols de bas poids moléculaire" signifie des diols présentant un poids moléculaire d'environ 62 à 700, de préférence de 62 à 200. Ils peuvent contenir des groupes aliphatiques, alicycliques ou aromatiques. Les composés préférés ne contiennent que des groupes aliphatiques. Les diols utilisés présentent de préférence jusqu'à 20 atomes de carbone et peuvent être choisi parmi l'éthylèneglycol, le diéthylèneglycol, le propane-1,2-diol, le propane-1,3-diol, le butane-1,4-diol, le butylène-1,3-glycol, le néopentylglycol, le butyléthylpropanediol, le cyclohexanediol, le 1,4-cyclohexanediméthanol, l'hexane-1,6-diol, le bisphénol A (2,2-bis(4-hydroxyphényl)propane), le bisphénol A hydrogéné (2,2-bis(4-hydroxycyclohexyl)propane), et leurs mélanges. De préférence, R₃ est issu du néopentylglycol.

Eventuellement, les diols de bas poids moléculaire peuvent contenir des groupes ioniques ou potentiellement ioniques. Des diols de bas poids moléculaire appropriés contenant des groupes ioniques ou potentiellement ioniques sont ceux divulgués dans le brevet US 3 412 054. Des composés préférés comprennent l'acide diméthylolbutanoïque (DMBA), l'acide diméthylolpropionique (DMBA) et le caprolactone-polyesterdiol contenant carboxyle. Si des diols de bas poids moléculaire contenant des groupes ioniques ou potentiellement ioniques sont utilisés, ils sont de préférence utilisés en une quantité telle que <0,30 méq de COOH est présent par gramme de polyuréthane dans la dispersion de polyuréthane. De préférence, les diols de bas poids moléculaire contenant des groupes ioniques ou potentiellement ioniques ne sont pas utilisés.

La chaîne du prépolymère est allongée en utilisant deux classes d'agent d'allongement de chaîne B) et C).

### B) Agents d'allongement de chaîne

Des composés B) de la première classe d'allongement de chaîne présentent la formule :

H₂N—R₄—NH₂

où R₄ représente un radical alkylène ou oxyde d'alkylène non substitué par des groupes ioniques ou potentiellement ioniques.

Ainsi, l'allongeur de chaîne peut être choisi parmi :
- Les alkylènediamines telles que l'hydrazine, l'éthylènediamine, la propylènediamine, la 1,4-butylènediamine et la pipérazine.
- Les diamines d'oxyde d'alkylène telles que le dipropylaminediéthylèneglycol (DPA-DEG disponible auprès de Tomah Products, Milton, WI), la 2-méthyl-1,5-pentanediamine (Dytec A de DuPont), l'hexanediamine, l'isophoronediamine, et la 4,4-méthylènedi(cyclohexylamine), et la série des DPA-étheramines disponibles chez Tomah Products, Milton, WI, comprenant le dipropylaminepropylèneglycol, le dipropylaminedipropylèneglycol, le dipropylaminetripropylèneglycol, le dipropylaminepoly(propylèneglycol), le dipropylaminéthylèneglycol, le dipropylaminepoly(éthylèneglycol), le dipropylamine-1,3-propanediol, le dipropylamine-2-méthyl-1,3-propanediol, le dipropylamine-1,4-butanediol, le dipropylamine-1,3-butanediol, le dipropylamine-1,6-hexanediol et le dipropylaminecyclohexane-1,4-diméthanol, et leurs mélanges.

De préférence, l'allongeur de chaîne B) est choisi parmi l'éthylènediamine, la diéthanolamine et leurs mélanges.

### C) Agents d'allongement de chaîne

La deuxième classe d'agents d'allongement de chaîne sont des composés C) présentant la formule :

H₂N—R₅-NH₂

où R₅ représente un radical alkylène substitué par des groupes ioniques ou potentiellement ioniques. Ces composés présentent un groupe ionique ou potentiellement ionique et deux groupes qui sont réactifs avec des groupes isocyanate. Le groupe ionique ou le groupe potentiellement ionique peut être choisi dans le groupe constitué par les groupes d'ammonium tertiaire ou quaternaire, les groupes convertibles en un tel groupe, un groupe carboxyle, un groupe carboxylate, un groupe acide sulfonique et un groupe sulfonate. Ladite conversion au moins partielle des groupes convertibles en groupes de sel du type mentionné peut avoir lieu avant ou pendant le mélange avec l'eau. Des composés spécifiques comprennent les diaminosulfonates, tels que par exemple le sel sodique de l'acide N-(2-aminoéthyl)-2-aminoéthanesulfonique (AAS) ou le sel sodique de l'acide N-(2-aminoéthyl)-2-aminopropionique.

De préférence, R₅ représente un radical alkylène substitué par des groupes d'acide sulfonique ou sulfonate.

De préférence, ce composé est le sel sodique de l'acide N-(2-aminoéthyl)-2-aminoéthanesulfonique (AAS).

### Terminaisons de chaîne

Le polyuréthane selon l'invention peut également comprendre des composés qui sont situés dans chaque cas aux extrémités des chaînes et terminent lesdites chaînes. Ces terminaisons de chaîne peuvent dériver de composés présentant la formule : 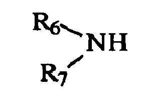 dans laquelle R₆ représente un atome d'hydrogène ou un radical alkylène présentant éventuellement une extrémité hydroxyle et R₇ représente un radical alkylène présentant éventuellement une extrémité hydroxyle. Des composés appropriés comprennent les composés tels que les monoamines, en particulier les monoamines secondaires ou les monoalcools. Des exemples comprennent : la méthylamine, l'éthylamine, la propylamine, la butylamine, l'octylamine, la laurylamine, la stéarylamine, l'isononyloxypropylamine, la diméthylamine, la diéthylamine, la dipropylamine, la dibutylamine, la N-méthylaminopropylamine, la diéthyl(méthyl)aminopropylamine, la morpholine, la pipéridine, la diéthanolamine et des dérivés substitués appropriés de celle-ci, les amide-amines de diamines primaires et d'acides monocarboxyliques, les monocétimes de diamines primaires, les amines primaires/tertiaires telles que la N,N-diméthylamino-propylamine et analogues. Les alcools de terminaison de chaîne peuvent être choisis parmi les alcools en C₁-C₁₀, tels que le méthanol, le butanol, l'hexanol, l'alcool 2-éthylhexylique, l'alcool isodécylique, et leurs mélanges, Les aminoalcools tels que l'aminométhylpropanol (AMP) conviennent également.

Dans une forme de réalisation de l'invention, le diéthylèneglycol est utilisé pour l'obtention du polyuréthane soit en tant que diol de bas poids moléculaire, soit comme partie de l'agent d'allongement de chaîne non ionique via l'utilisation de dipropylaminediéthylèneglycol. Dans le cas où le diéthylèneglycol est utilisé comme diol de bas poids moléculaire, alors, de préférence, le DPA-DEG n'est pas utilisé comme agent d'allongement de chaîne non ionique. De manière analogue, si le DPA-DEG est utilisé comme agent d'allongement de chaîne non ionique, alors, de préférence, on n'utilise pas de diéthylèneglycol comme diol de bas poids moléculaire.

### Procédés de préparation

Les compositions selon l'invention comprennent une dispersion aqueuse de polyuréthane appropriée pour une utilisation dans les produits de maquillage ou de soins des fibres kératiniques telles que les cils ou sourcils, et sont susceptibles d'être obtenues par un procédé de préparation comprenant les étapes suivantes :
A) la préparation d'une dispersion aqueuse de polyuréthane par
   1) la formation d'un prépolymère à fonctionnalité isocyanate en faisant réagir :
      1a) un polyesterpolyol, et notamment un polyesterdiol,
      1b) un polyisocyanate aliphatique ou cycloaliphatique, et
      1c) un diol de bas poids moléculaire, éventuellement substitué par des groupes ioniques ;
   2) un allongement de chaîne du prépolymère par :
      2a) au moins un agent d'allongement de chaîne selon la formule :
         H₂N-R₄-NH₂
            où R₄ représente un radical alkylène ou oxyde d'alkylène non substitué par des groupes ioniques ou potentiellement ioniques, et
      2b) au moins un agent d'allongement de chaîne selon la formule :
         H₂N-R₅-NH₂
            où R₅ représente un radical alkylène substitué par des groupes ioniques ou potentiellement ioniques,
            en présence d'un solvant organique pour former un polyuréthane ;
      3) la dispersion du polyuréthane dans l'eau ; et
      4) l'élimination du solvant organique, ce qui permet d'obtenir une dispersion aqueuse de polyuréthane ; et
         le mélange de la dispersion de polyuréthane avec de l'eau ou de l'éthanol

Plus particulièrement, un procédé de production d'une dispersion de polyuréthane appropriée pour une utilisation dans des produits de maquillage, peut comprendre les étapes suivantes : a) la réaction dans une première étape d'au moins un composé polyesterpolyol et d'un diol de bas poids moléculaire éventuellement substitué par un groupe ionique (des composés dihydroxyle) avec du diisocyanate pour former le prépolymère A), puis b) la dissolution dans une deuxième étape du prépolymère dans un solvant organique et c) la réaction dans une troisième étape de la solution de prépolymère contenant l'isocyanate avec les deux classes d'agents d'allongement de chaîne et, éventuellement, la terminaison de chaîne, d) la formation, dans une quatrième étape, de la dispersion par addition d'eau, et e) l'élimination, dans une cinquième étape, du solvant organique.

Les groupes d'acide sulfonique libres incorporés sont neutralisés entre la troisième et la quatrième étape. Des agents de neutralisation appropriés compris sont les amines primaires, secondaires ou tertiaires. Parmi ceux-ci, on préfère les amines tertiaires substituées par trialkyle. Des exemples de ces amines sont la triméthylamine, la triéthylamine, la triisopropylamine, la tributylamine, la N,N-diméthylcyclohexylamine, la N,N-diméthylstéarylamine, la N,N-diméthylaniline, la N-méthylmorpholine, la N-éthylmorpholine, la N-méthylpipérazine, la N-méthylpyrrolidine, la N-méthylpipéridine, la N,N-diméthyléthanolamine, la N,N-diéthyléthanolamine, la triéthanolamine, la N-méthyldiéthanolamine, le diméthylaminopropanol, la 2-méthoxyéthyldiméthylamine, la N-hydroxyéthylpipérazine, le 2-(2-diméthylaminoéthoxy)-éthanol et la 5-diéthylamino-2-pentanone. Les amines tertiaires préférées sont celles qui ne contiennent pas d'hydrogène(s) actif(s) comme déterminé par le test de Zerewitinoff étant donné que l'hydrogène peut réagir avec les groupes isocyanate des prépolymères ce qui peut provoquer une gélification, la formation de particules insolubles ou la terminaison des chaînes.

Les dispersions de polyuréthane peuvent être produites par ce qu'on appelle le procédé à l'acétone. Dans le procédé à l'acétone, la synthèse des préparations aqueuses de polyuréthane à la base des dispersions selon l'invention est réalisée dans un procédé à étapes multiples.

Dans une première étape, un prépolymère contenant des groupes isocyanate est synthétisé à partir du composé polyesterpolyol, du diisocyanate et du diol de bas poids moléculaire. Les quantités de composants individuels sont calculées de manière telle que la teneur en isocyanate des prépolymères est située entre 1,4 et 5,0% en poids, de préférence entre 2,0 et 4,5% en poids, et de manière particulièrement préférée entre 2,6 et 4,0% en poids. Le diol de bas poids moléculaire est présent en une quantité de 0 à 80% en équiv sur base de la quantité d'équivalents NCO, de préférence de 0 à 10% en équiv.

Le prépolymère obtenu présente la structure : 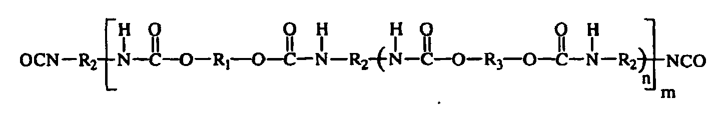 où
R₁ représente un polyesterpolyol et notamment un polyesterdiol,
R₂ représente un radical hydrocarboné ou un polyisocyanate aliphatique ou cycloaliphatique,
R₃ représente un radical d'un diol de bas poids moléculaire, éventuellement substitué par des groupes ioniques,
n vaut <5, et
m vaut > 1.

De préférence, n vaut 1 à 3, et m vaut 1 à 5.

Dans une deuxième étape, le prépolymère produit dans l'étape 1 est dissout dans un solvant organique, au moins partiellement miscible à l'eau, ne contenant pas de groupes réactifs avec isocyanate. Le solvant préféré est l'acétone. D'autres solvants tels que par exemple la 2-butanone, le tétrahydrofuranne ou le dioxane ou les mélanges de ces solvants peuvent cependant aussi être utilisés. Les quantités de solvant à utiliser doivent être calculées de manière telle qu'une teneur en solides de 25 à 60% en poids, de préférence de 30 à 50% en poids, de manière particulièrement préférée de 35 à 45% en poids, est obtenue.

Dans une troisième étape, on fait réagir la solution de prépolymère contenant isocyanate avec des mélanges d'agents d'allongement de chaîne à fonctionnalité amino et, éventuellement, des terminaisons de chaîne, pour former le polyuréthane de poids moléculaire élevé. Des quantités suffisantes d'agents d'allongement de chaîne et de terminaison de chaîne sont utilisées de manière telle que le poids moléculaire numérique moyen calculé (Mn) du polyuréthane obtenu est situé entre 10 000 et 100 000 Daltons, de préférence entre 10 000 et 50 000 Daltons. L'agent d'allongement de chaîne non ionique est présent en une quantité de 15 à 90% en équiv, de préférence de 35,0 à 55% en équiv, sur base de la quantité résiduelle d'équivalents NCO présents dans le prépolymère. L'agent d'allongement de chaîne ionique est présent en une quantité de 10 à 50% en équiv, de préférence de 25 à 35% en équiv, sur base de la quantité résiduelle d'équivalents NCO présents dans le prépolymère. La terminaison de chaîne est présente en une quantité de 0 à 35% en équiv, de préférence de 20 à 30% en équiv, sur base de la quantité résiduelle d'équivalents NCO présents dans le prépolymère.

Dans une quatrième étape, le polyuréthane de poids moléculaire élevé est dispersé sous forme d'une dispersion à fines particules par l'addition d'eau à la solution ou de la solution à l'eau.

Dans une cinquième étape, le solvant organique est partiellement ou totalement éliminé par distillation, éventuellement sous pression réduite. La quantité d'eau dans l'étape quatre est calculée de manière telle que les dispersions aqueuses de polyuréthane selon l'invention affichent une teneur en solides de 20 à 60% en poids, de préférence de 28 à 42% en poids.

### PHASE AQUEUSE

La composition conforme à l'invention peut comprendre une phase aqueuse comprenant de l'eau et/ou au moins un solvant hydrosoluble. Cette phase aqueuse est continue.

Par composition à phase continue aqueuse, on entend que la composition présente une conductivité, mesurée à 25 °C, supérieure ou égale à 23 S/cm (microSiemens/cm), la conductivité étant mesurée par exemple à l'aide d'un conductimètre MPC227 de Mettler Toledo et d'une cellule de mesure de conductivité Inlab730. La cellule de mesure est immergée dans la composition, de façon à éliminer les bulles d'air susceptibles de se former entre les 2 électrodes de la cellule. La lecture de la conductivité est faite dès que la valeur du conductimètre est stabilisée. Une moyenne est réalisée sur au moins 3 mesures successives.

Par "solvant hydrosoluble", on désigne dans la présente invention un composé liquide à température ambiante et miscible à l'eau (miscibilité dans l'eau supérieure à 50 % en poids à 25 °C et pression atmosphérique).

Les solvants hydrosolubles utilisables dans les compositions selon l'invention peuvent en outre être volatils.

Parmi les solvants hydrosolubles pouvant être utilisés dans les compositions conformes à l'invention, on peut citer notamment les monoalcools inférieurs ayant de 1 à 5 atomes de carbone tels que l'éthanol et l'isopropanol, les glycols ayant de 2 à 8 atomes de carbone tels que l'éthylène glycol, le propylène glycol, le 1,3-butylène glycol et le dipropylène glycol, les cétones en C₃ et C₄ et les aldéhydes en C₂-C₄.

La phase aqueuse (eau et éventuellement le solvant miscible à l'eau) peut être présente dans la composition en une teneur allant de 1 % à 95 % en poids, par rapport au poids total de la composition, de préférence allant de 5 % à 80 % en poids, et préférentiellement allant de 10 % à 60 % en poids.

La phase aqueuse selon l'invention peut également comprendre au moins un polymère filmogène hydrophile et/ou au moins un épaississant hydrophile et/ou au moins un tensioactif, tel que ceux listés précédemment. Toutefois, la teneur en phase aqueuse précédemment indiquée n'inclut pas les teneurs de chacun des composés suscités.

### AGENT PLASTIFIANT

La composition conforme à l'invention peut comprendre un agent plastifiant favorisant la formation d'un film avec le polymère filmogène, et ce notamment avec le polyuréthane mis en oeuvre dans la présente invention. L'agent plastifiant vise notamment à rendre plus flexible ledit polyuréthane, et ce en diminuant sa température de transition vitreuse (Tg).

En particulier le plastifiant peut être avantageusement choisi parmi les agents plastifiants lipophiles.

Par agent plastifiant lipophile, on désigne dans la présente invention un composé liquide à température ambiante (25°C), présentant de préférence une viscosité inférieure à 100cPs, de préférence inférieure à 50cPs, et présentant une solubilité dans l'eau à 25°C inférieure à 10%, de préférence inférieure à 5%.

L'agent plastifiant est en particulier choisi parmi les (poly)esters issus de la ou des réaction(s) d'au moins un acide carboxylique avec au moins un (poly)ol, les éthers de glycol, le N-éthyl-o,p-toluènesulfonamide, les carbonates, les cétones, et leurs mélanges.

Par (poly)ol on entend un composé polyol ou monoalcool.

Par polyol on entend un composé organique comprenant au moins 2 groupes hydroxyles, particulièrement entre 2 et 6 groupes hydroxyles, lesdits groupes hydroxyles étant portés par un groupe i) alkyle en C1-C6 éventuellement interrompu par de 1 à 6 hétéroatomes choisi parmi N, S, 0 ; ii) aryle C5-C20, iii) hétéroaryle comprenant entre 5 et 20 chaînons avec 1 à 3 hétéroatome(s) choisi(s) parmi N, S, O ; iv) hétérocycloalkyle ; et v) cycloalkyle. En particulier, à titre de poly(ol), on peut par exemple citer l'éthylène glycol ou le glycérol.

Par monoalcool, on entend un composé organique comprenant un unique groupe hydroxyle, ledit groupe hydroxyle étant porté par un groupe i) alkyle en C1-C6 éventuellement interrompu par de 1 à 6 hétéroatomes choisi parmi N, S, O ; ii) aryle C5-C20 ; iii) hétéroaryle comprenant entre 5 et 20 chaînons avec 1 à 3 hétéroatome(s) choisi(s) parmi N, S, O ; iv) hétérocycloalkyle ; et v) cycloalkyle.

L'agent plastifiant peut ainsi être choisi plus particulièrement parmi :
a) Les esters issus de la réaction, et notamment de la monocondensation ou de la polycondensation, d'un ou plusieurs acides carboxyliques ou orthophosphorique avec un ou plusieurs (poly)ol, tel qu'un diol. De tels esters peuvent être des (mono/poly)esters, et de préférence des polyesters, tels que des diesters.
   De tels esters peuvent de façon générale être, issus de la réaction d'un ou plusieurs acide(s) (mono/poly)carboxylique de formule R₁₁(COOH)n avec un ou plusieurs (poly)ol de formule R₁₂(OH)m avec :
   - R₁₁ et R₁₂, identiques ou différents, représentent une chaîne hydrocarbonée, comprenant de préférence de 3 à 15 atomes de carbone, linéaire, ramifiée ou cycloalkyle en C3-C15 cyclique, saturée ou insaturée, éventuellement non aromatique, comprenant éventuellement un ou plusieurs hétéroatomes tels que N, O, S tel que de 0 à 6 hétéroatomes,
   - n allant de 1 à 6, et
   - m allant de 1 à 6
   De préférence, R₁₁ est un radical alkyle en C₂-C₅ tel que l'éthyle, le propyle, le butyle, l'isobutyle et R₁₂ est une chaîne hydrocarbonée linéaire saturée ou ramifiée comprenant de 5 à 10 atomes de carbone.
   A titre d'exemples d'esters, on peut en particulier citer :
   - des esters issus de la réaction de R(COOH) où R représente une chaîne alkyle ramifiée de C3 à C10 tel que l'isobutyle ou le terbutyle, avec HOC(R)(R')-C(OH)-CH(R")-R"' où R représente un atome d'hydrogène ou une chaîne alkyle linéaire ou ramifiée tel que méthyle et R', R", R"' représentant une chaîne alkyle linéaire ou ramifiée tel que méthyle. On peut notamment citer les esters issus de la monocondensation ou de la dicondensation de l'acide tertio-butylique et du tri-méthyl-2,2,4 pentane-diol-1,3. En particulier, on peut citer un monoester résultant de la réaction de l'acide isobutyrique et d'octanediol tel que le triméthyl-2,2,4 pentane diol 1,3, comme le TEXANOL Ester Alcohol commercialisé par la société Eastman Chemical.
   - des esters d'acide phosphorique issus de la condensation entre un ou plusieurs (poly)ol et un ou plusieurs acide orthophosphorique de formule (R-Z)n-P=O(O-R')ₘ avec R, R' représentant un groupe alkyle, linéaire ou ramifié, en C₁-C₂₀, n étant égal à 1, 2 ou 3 et m étant égal à 0, 1 ou 2, avec m + n étant égal à 3 et Z un hétéroatome tel que l'oxygène ou une liaison sigma. A titre d'exemple de tels esters on peut citer entre autre le phosphate de tricrésyle, le phosphate de tributyle, le phosphate de triphényle, le phosphate de tributoxyéthyle ;
   - des esters d'acide gras issus de la (mono/poly)condensation entre un ou plusieurs (poly)ol et un ou plusieurs acides gras de formule R(COOH)n avec R étant une chaîne alkyle linéaire saturée de C3 à C22 et n étant égal à 1 ou 2. A titre d'exemple de tels esters on peut citer entre autre les esters d'acide adipique, tels que l'adipate de diisobutyle, l'adipate de diéthyle ou des esters d'acide stéarique, tels que le stéarate d'éthyle, ou encore des esters d'acide palmitique, tels que le palmitate de 2-éthyl hexyle.
   - des esters d'acide citrique ou citrate, tels que le citrate de triéthyle, le citrate de tributyle, l'acétylcitrate de triéthyle, l'acétylcitrate de tributyle, l'acétylcitrate de triéthyl-2 hexyle ;
   - des esters d'acide phtalique ou phtalate, tels que le phtalate de diéthyle, le phtalate de dibutyle, le phtalate de dioctyle, le phtalate de dipentyle, le phtalate de diméthoxyéthyle, le phtalate de butyle et de 2-éthyl hexyle ;
   - des esters d'acide tartrique ou tartrates, notamment le tartrate de dibutyle ;
   - des esters d'acide sébacique ou sébaçates tels que le sébaçate de diméthyle, le sébaçate de dibutyle ;
   - le diacétate de propylène glycol et le triacétate de glycérol,
   - le benzoate de benzyle,
   - l'acétylricinoléate de butyle, l'acétylricinoléate de glycéryle, et
   - le glycolate de butyle.
b) Les éthers de glycol
   A titre d'exemples d'éthers de glycol, on peut citer le diéthylène glycol éthyléther, le diéthylène glycol méthyléther, le diéthylène glycol butyléther, le diéthylène glycol hexyléther, l'éthylène glycol éthyléther, l'éthylène glycol butyléther, l'éthylène glycol hexyléther ou encore le propylène glycol phényléther, le dipropylène glycol butyléther, le tripropylène glycol butyléther, le propylène glycol méthyléther, le dipropylène glycol éthyléther, le tripropylène glycol méthyléther, le diéthylène glycol méthyléther et le propylène glycol butyléther,
c) On peut également utiliser comme agent plastifiant le N-éthyl-o,p-toluènesulfonamide et en particulier l'éthyltosylamide tel que commercialisé sous la référence RESIMPOL 8 par la société Pan-Americana.
d) Les carbonates ;
e) Les cétones, notamment le camphre,
   et leurs mélanges.

Le ou les agents plastifiants peu(ven)t être présent(s) en une quantité allant de 1 à 15% en poids par rapport au poids total de la composition, de préférence de 2 à 10%, et mieux de 3 à 8% en poids. De manière générale cet agent plastifiant peut être présent à une quantité en poids supérieure ou égale, mieux strictement supérieure, à 2% par rapport au poids total de la composition.

### TENSIOACTIFS

La composition selon l'invention peut contenir un système émulsionnant comportant un ou plusieurs agents tensioactifs présents notamment en une teneur allant de 0.1% à 20% en poids par rapport au poids total de la composition, voire 0,5% à 15% en poids, de préférence allant de 1 % à 10% en poids.

On utilise généralement un agent tensioactif émulsionnant choisi de manière appropriée pour l'obtention d'une émulsion huile-dans-eau. En particulier, on peut utiliser un agent tensioactif émulsionnant possédant à 25 °C une balance HLB (hydrophile-lipophile balance) au sens de GRIFFIN, supérieure ou égale à 8.

La valeur HLB selon GRIFFIN est définie dans J. Soc. Cosm. Chem. 1954 (volume 5), pages 249-256.

Ces agents tensioactifs peuvent être choisis parmi des agents tensioactifs non ioniques, anioniques, cationiques, amphotères et leurs mélanges. On peut se reporter au document « Encyclopedia of Chemical Technology, KIRK-OTHMER », volume 22, p. 333-432, 3ème édition, 1979, WILEY, pour la définition des propriétés et des fonctions émulsionnantes des agents tensioactifs, en particulier p. 347-377 de cette référence, pour les agents tensioactifs anioniques, amphotères et non ioniques.

Les agents tensioactifs utilisés préférentiellement dans la composition selon l'invention sont choisis parmi :
a) les agents tensioactifs non ioniques de HLB supérieur ou égal à 8 à 25 °C, utilisés seuls ou en mélange; on peut citer notamment :
   - les éthers de glycérol, oxyéthylénés et/ou oxypropylénés, pouvant comporter de 1 à 150 motifs oxyéthylène et/ou oxypropylène ;
   - les alcools oxyalkylénés, en particulier oxyéthylénés et/ou oxypropylénés, pouvant comporter de 1 à 150 motifs oxyéthylène et/ou oxypropylène, de préférence de 20 à 100 motifs oxyéthylène, en particulier lesalcools gras, notamment en C₈-C₂₄, et de préférence en C₁₂-C₁₈ , éthoxylés tels que l'alcool stéarylique éthoxylé à 20 motifs oxyéthylène (nom CTFA "Steareth-20 ") comme le BRIJ 78 commercialisé par la société UNIQEMA, l'alcool cétéarylique éthoxylé à 30 motifs oxyéthylène (nom CTFA "Ceteareth-30 ") et le mélange d'alcools gras en C₁₂-C₁₅ comportant 7 motifs oxyéthylène (nom CTFA "C₁₂-₁₅ Pareth-7") comme celui commercialisé sous la dénomination NEODOL 25-7® par SHELL CHEMICALS ;
   - les esters d'acide gras, notamment en C₈-C₂₄, et de préférence en C₁₆-C₂₂, et de polyéthylène glycol (ou PEG) (pouvant comprendre de 1 à 150 motifs oxyéthylène), tels que le stéarate de PEG-50 et le monostéarate de PEG-40 commercialisé sous le nom MYRJ 52P® par la société UNIQEMA ;
   - les esters d'acide gras, notamment en C₈-C₂₄, et de préférence en C₁₆-C₂₂, et d'éthers de glycérol oxyéthylénés et/ou oxypropylénés (pouvant comporter de 1 à 150 motifs oxyéthylène et/ou oxypropylène), comme le monostéarate de glycéryle polyoxyéthyléné à 200 motifs oxyéthylène, vendu sous la dénomination Simulsol 220 TM@ par la société SEPPIC ; le stéarate de glycéryle polyoxyéthyléné à 30 motifs oxyéthylène comme le produit TAGAT S® vendu par la société GOLDSCHMIDT, l'oléate de glycéryle polyoxyéthyléné à 30 motifs oxyéthylène comme le produit TAGAT O® vendu par la société GOLDSCHMIDT, le cocoate de glycéryle polyoxyéthyléné à 30 motifs oxyéthylène comme le produit VARIONIC LI 13® vendu par la société SHEREX, l'isostéarate de glycéryle polyoxyéthyléné à 30 motifs oxyéthylène comme le produit TAGAT L®vendu par la société GOLDSCHMIDT et le laurate de glycéryle polyoxyéthyléné à 30 motifs oxyéthylène comme le produit TAGAT 1® de la société GOLDSCHMIDT ;
   - les esters d'acide gras, notamment en C₈-C₂₄, et de préférence en C₁₆-C₂₂, et d'éthers de sorbitol oxyéthylénés et/ou oxypropylénés (pouvant comporter de 1 à 150 motifs oxyéthylène et/ou oxypropylène), comme le polysorbate 60 vendu sous la dénomination Tween 60® par la société UNIQEMA ;
   - le diméthicone copolyol, tel que celui vendu sous la dénomination Q2-5220® par la société DOW CORNING ;
   - le diméthicone copolyol benzoate tel que celui vendu sous la dénomination FINSOLV SLB 101® et 201® par la société FINTEX ;
   - les copolymères d'oxyde de propylène et d'oxyde d'éthylène, également appelés polycondensats OE/OP,
   et leurs mélanges.
   Les polycondensats OE/OP sont plus particulièrement des copolymères consistant en des blocs polyéthylène glycol et polypropylène glycol, comme par exemple les polycondensats tribloc polyéthylène glycol/polypropylène glycol/polyéthylène glycol. Ces polycondensats tribloc ont par exemple la structure chimique suivante :

   H-(O-CH₂-CH₂)ₐ-(O-CH(CH₃)-CH₂)_{b}-(O-CH₂-CH₂)ₐ-OH,

   formule dans laquelle a va de 2 à 120, et b va de 1 à 100.
   Les polycondensats OE/OP ont de préférence une masse moléculaire moyenne en poids allant de 1 000 à 15 000, et mieux allant de 2 000 à 13 000. Avantageusement, lesdits polycondensats OE/OP ont une température de trouble, à 10 g/l en eau distillée, supérieure ou égale à 20 °C, de préférence supérieure ou égale à 60 °C. La température de trouble est mesurée selon la norme ISO 1065. Comme polycondensat OE/OP utilisable selon l'invention, on peut citer les polycondensats tribloc polyéthylène glycol/polypropylène glycol/ polyéthylène glycol vendus sous les dénominations SYNPERONIC® comme les SYNPERONIC PE/ L44® et SYNPERONIC PE/F127® par la société ICI.
b) les agents tensioactif non ioniques de HLB inférieur à 8 à 25 °C, éventuellement associés à un ou plusieurs agents tensioactif non ioniques de HLB supérieur à 8 à 25 °C, tels que cités ci-dessus, comme :
   - les esters et éthers d'oses tels que les stéarate de saccharose, cocoate de saccharose, stéarate de sorbitan et leurs mélanges, par exemple l'Arlatone 2121® commercialisé par la société ICI ou le SPAN 65V de la société UNIQEMA ;
   - les esters d'acides gras, notamment en C₈-C₂₄, et de préférence en C₁₆-C₂₂, et de polyol, notamment de glycérol ou de sorbitol, tels que le stéarate de glycéryle, par exemple vendu sous la dénomination TEGIN M® par la société GOLDSCHMIDT, le laurate de glycéryle tel que le produit vendu sous la dénomination IMWITOR 312® par la société HULS, le stéarate de polyglycéryle-2, le tristéarate de sorbitan, et le ricinoléate de glycéryle ;
   - les alcools oxyalkylénés, en particulier oxyéthylénés et/ou oxypropylénés pouvant comporter de 1 à 15 motifs oxyéthylène et/ou oxypropylène, en particulier les alcools gras en C₈-C₂₄, et de préférence en C₁₂-C₁₈ , éthoxylés tels que l'alcool stéarylique éthoxylé à 2 motifs oxyéthylène (nom CTFA "Steareth-2 ") tel que le BRIJ 72 commercialisé par la société UNIQEMA ;
   - le mélange de cyclométhicone/diméthicone copolyol vendu sous la dénomination Q2-3225C® par la société DOW CORNING ;
c) les tensioactifs anioniques tels que :
   - les sels d'acides gras en C₁₆-C₃₀, notamment les sels aminés comme le stéarate de triéthanolamine ou le stéarate de 2-amino-2-méthylpropane-1,3-diol ;
   - les sels d'acides gras polyoxyéthylénés, notamment les sels aminés ou les sels de métaux alcalins, et leurs mélanges ;
   - les esters phosphoriques et leurs sels tels que le "DEA oleth-10 phosphate" (Crodafos N 10N de la société CRODA) ou le phosphate de monocétyle monopotassique (Amphisol K de Givaudan ou ARLATONE MAP 160K de la société UNIQEMA) ;
   - les sulfosuccinates tels que le "Disodium PEG-5 citrate lauryl sulfosuccinate" et le "Disodium ricinoleamido MEA sulfosuccinate" ;
   - les alkyléthersulfates tels que le lauryl éther sulfate de sodium ;
   - les iséthionates ;
   - les acylglutamates tels que le "Disodium hydrogenated tallow glutamate" (AMISOFT HS-21 R® commercialisé par la société AJINOMOTO) et leurs mélanges.

A titre d'exemples de tensioactif cationique, on peut notamment citer :
- les alkylimidazolidiniums tels que l'étho-sulfate d'isostéaryléthylimidonium,
- les sels d'ammonium tels que les halogénures d'(alkyl en C₁₂₋₃₀)-tri(alkyl en C ₁₋₄)ammonium comme le chlorure de N,N,N-triméthyl-1-docosanaminium (ou chlorure de Behentrimonium).

Les compositions selon l'invention peuvent également contenir un ou plusieurs tensioactifs amphotères comme les N-acyl-aminoacides tels que les N-alkyl-aminoacétates et le cocoamphodiacetate disodique, et les oxydes d'amines tels que l'oxyde de stéaramine, ou encore des tensioactifs siliconés comme les diméthicone copolyols phosphates tels que celui vendu sous la dénomination PECOSIL PS 100® par la société PHOENIX CHEMICAL.

Selon un mode de réalisation particulier de l'invention le système émulsionnant peut comprendre au moins un agent tensioactif choisi parmi :
i) un alkyl phosphate de métal alcalin de formule ou oxyde de phosphine (R-O)n-P=O(O⁻M)ₘ avec R représentant un groupe alkyle, linéaire ou ramifié, en C₈-C₂₂ tel que le cétyle, n étant égal à 1, 2 ou 3 et m étant égal à 0, 1 ou 2, avec m + n étant égal à 3 et M représentant un atome d'hydrogène ou un métal alcalin ou alcalino terreux, préférentiellement n = 1 et m= 2, et M est un métal alcalin, tel que le sodium ou potassium,
ii) un alcool polyéthoxylé de formule R'-(OCH₂CH₂)p-OH avec R' représentant un alkyle linéaire ou ramifié en C₁-C₃₀ , de préférence en C8-C24, mieux en en C₁₂-C₁₈ et particulièrement représente CH₃-(CH₂)₁₇- et p représentant un entier compris inclusivement entre 2 et 30, préférentiellement entre 2 et 20 ; tels que le steareth-20, le steareth-2,
iii) un sel d'acide glutamique de formule R-CONH-C(COO-M)-C₂H₄-COO-M' avec R représentant un groupe alkyle, linéaire ou ramifié en C₈-C₂₂ tel que le stéaryle et M' représentant un métal alcalin ou alcalino terreux et
iv) un alkyl glucoside obtenu par condensation de glucose et d'alcools gras linéaires ou ramifiés en C₈-C₂₂ tel qu'un mélange cetylique et stéarique nommé cétylstéaryle.

En particulier, le système émulsionnant peut comprendre au moins un agent tensioactif choisi parmi le cétyl phosphate de potassium, le steareth 2, le steareth 20 et leurs mélanges. En variante ou de façon additionnelle, ce système émulsionnant peut comprendre au moins un agent tensioactif choisi parmi le glutamate de stéaroyle de sodium et le glucoside.de cétylstearyle, et leurs mélanges.

Selon l'invention ou selon un mode de réalisation avantageux, selon l'objet de l'invention considéré, la composition de maquillage comprend moins de 1 % en poids de triéthanolamine par rapport au poids total de la composition, de préférence moins de 0,5 % voire moins de 0.1 %, ou est exempte de triéthanolamine.

### Polymère filmogène hydrophile

La composition de maquillage peut comprendre, outre le polyuréthane particulier de la présente invention, au moins un polymère filmogène additionnel. Ce polymère filmogène additionnel peut être lipophile ou hydrophile.

On entend par "polymère filmogène", un polymère apte à former à lui seul ou en présence d'un agent auxiliaire de filmification, un film macroscopiquement continu et adhérent sur les cils, et de préférence un film cohésif, et mieux encore un film dont la cohésion et les propriétés mécaniques sont telles que ledit film peut être isolable et manipulable isolément, par exemple lorsque ledit film est réalisé par coulage sur une surface anti-adhérente comme une surface téflonnée ou siliconnée.

De façon générale, la teneur en matières sèches en polymère filmogène additionnel dans la composition peut varier de 0,1 à 40 %, de préférence de 0,5 à 30 % et mieux de 1 à 10 % en poids, par rapport au poids total de la composition.

Parmi les polymères filmogènes utilisables dans la composition de la présente invention, on peut citer les polymères synthétiques, de type radicalaire ou de type polycondensat, les polymères d'origine naturelle, et leurs mélanges.

Le polymère filmogène hydrophile peut être un polymère hydrosoluble ou se présenter en dispersion dans un milieu aqueux.

Comme exemples de polymères filmogènes hydrosolubles, on peut citer :
- les protéines comme les protéines d'origine végétale telles que les protéines de blé, de soja ; les protéines d'origine animale tels que les kératines, par exemples les hydrolysats de kératine et les kératines sulfoniques ;
- les polymères de cellulose tels que l'hydroxyéthylcellulose, l'hydroxypropylcellulose, la méthylcellulose, l'éthylhydroxyéthylcellulose, la carboxyméthylcellulose, ainsi que les dérivés quaternisés de la cellulose ;
- les polymères ou copolymères acryliques, tels que les polyacrylates ou les polyméthacrylates ;
- les polymères vinyliques, comme les polyvinylpyrrolidones, les copolymères de l'éther méthylvinylique et de l'anhydride malique, le copolymère de l'acétate de vinyle et de l'acide crotonique, les copolymères de vinylpyrrolidone et d'acétate de vinyle ; les copolymères de vinylpyrrolidone et de caprolactame ; l'alcool polyvinylique ;
- les polymères de chitine ou de chitosane anioniques, cationiques, amphotères ou non-ioniques,
- les gommes arabiques, la gomme de guar, les dérivés du xanthane, la gomme de karaya ;
- les alginates et les carraghénanes ;
- les glycoaminoglycanes, l'acide hyaluronique et ses dérivés ;
- la résine shellac, la gomme de sandaraque, les dammars, les élémis, les copals ;
- l'acide désoxyribonucléïque ;
- les muccopolysaccharides tels les chondroïtines sulfate,
- et leurs mélanges.

Le polymère filmogène peut être également présent dans la composition sous la forme de particules en dispersion dans une phase aqueuse, connue généralement sous le nom de latex ou pseudolatex. Les techniques de préparation de ces dispersions sont bien connues de l'homme du métier.

Comme dispersion aqueuse de polymère filmogène, on peut utiliser les dispersions acryliques vendues sous les dénominations Neocryl XK-90^{®}, Neocryl A-1070^{®}, Neocryl A-1090^{®}, Neocryl BT-62^{®}, Neocryl A-1079^{®} et Neocryl A-523^{®}par la société AVECIA-NEORESINS, Dow Latex 432^{®} par la société DOW CHEMICAL, Daitosol 5000 AD^{®} ou Daitosol 5000 SJ^{®} par la société DAITO KASEY KOGYO; Syntran 5760^{®} par la société Interpolymer Allianz Opt^{®}par la société Rohm and Haas ou encore les dispersions aqueuses de polyuréthane vendues sous les dénominations Neorez R-981^{®} et Neorez R-974^{®} par la société AVECIA-NEORESINS, les Avalure UR-405^{®}, Avalure UR-410^{®}, Avalure UR-425^{®}, Avalure UR-450^{®}, Sancure 875^{®}, Avalure UR-445^{®}et Sancure 2060^{®} par la société NOVEON, Impranil 85^{®} par la société BAYER, Aquamere H-1511^{®} par la société HYDROMER ; les sulfopolyesters vendus sous le nom de marque Eastman AQ^{®} par la société EASTMAN CHEMICAL PRODUCTS, les dispersions vinyliques comme le Mexomère PAM^{®}, les dispersions aqueuses de polyvinyl acétate comme le "Vinybran^{®}" de la société Nisshin Chemical ou celles commercialisées par la société UNION CARBIDE, les dispersions aqueuses de terpolymère vinyl pyrrolidone, diméthylaminopropyl méthacrylamide et chlorure de lauryldiméthylpropylméthacrylamidoammonium telles que le Styleze W-d'ISP, les dispersions aqueuses de polymères hybrides polyuréthane/polyacryliques telles que celles commercialisées sous les références "Hybridur^{®}" par la société AIR PRODUCTS ou "Duromer^{®}" de NATIONAL STARCH, les dispersions type core/shell : par exemple celles commercialisées par la société ATOFINA sous la référence Kynar (core : fluoré- shell : acrylique) ou encore ceux décrits dans le document US 5 188 899 (core ; silice - shell : silicone) et leurs mélanges.

Selon un mode particulier de réalisation, la composition de l'invention peut comprendre à titre de polymères filmogènes hydrophiles au moins l'association d'un polymère cationique et d'un polymère anionique.

Le polymère cationique peut être choisi parmi les dérivés d'éther de cellulose quaternaires, les copolymères de cellulose avec un monomère hydrosoluble d'ammonium quaternaire, les cyclopolymères, les polysaccharides cationiques, les polymères cationiques siliconés, les copolymères vinylpyrrolidone-acrylate ou -méthacrylate de dialkylamino-alkyle quaternisés ou non, les polymères quaternaires de vinylpyrrolidone et de vinylimidazole, les polyamidoamines et leurs mélanges.

De préférence, le polymère cationique est une hydroxyalkyl(C₁-C₄)Cellulose comportant des groupements ammonium quaternaires.

Le polymère anionique est avantageusement choisi parmi :
A) les homo- ou copolymères d'acide acrylique ou méthacrylique ou leurs sels, les copolymères d'acide acrylique et d'acrylamide et leurs sels, les sels de sodium d'acides polyhydroxycarboxyliques tels que les copolymères d'acide acrylique et d'acrylamide vendus sous la forme de leur sel de sodium sous les dénominations RETEN^{®} par la société HERCULES, le polyméthacrylate de sodium vendu sous la dénomination DARVAN N°7^{®} par la société VANDERBILT, les sels de sodium d'acides polyhydroxycarboxyliques vendus sous la dénomination HYDAGEN F^{®} par la société HENKEL,
B) les copolymères des acides acrylique ou méthacrylique avec un monomère monoéthylénique tel que l'éthylène, le styrène, les esters vinyliques, les esters d'acide acrylique ou méthacrylique, éventuellement greffés sur un polyalkylène glycol tel que le polyéthylène glycol; les copolymères de ce type comportant dans leur chaîne un motif acrylamide éventuellement N-alkylé et/ou hydroxyalkylé, les copolymères d'acide acrylique et de méthacrylate d'alkyle en C1-C4 et les terpolymères de vinylpyrrolidone, d'acide acrylique et de méthacrylate d'alkyle en C1-C20 ;
C) les copolymères dérivés d'acide crotonique tels que ceux comportant dans leur chaîne des motif acétate ou propionate de vinyle et éventuellement d'autres monomères tels que esters allylique ou méthallylique, éther vinylique ou ester vinylique d'un acide carboxylique saturé linéaire ou ramifié à longue chaîne hydrocarbonée tels que ceux comportant au moins 5 atomes de carbone, ces polymères pouvant éventuellement être greffés ;
D) les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters ; les copolymères d'anhydrides maléique, citraconique, itaconique et d'un ester allylique ou méthallylique comportant éventuellement un groupement acrylamide, méthacrylamide, une α-oléfine, des esters acryliques ou méthacryliques, des acides acrylique ou méthacrylique ou la vinylpyrrolidone dans leur chaîne, les fonctions anhydrides sont monoestérifiées ou monoamidifiées ;
E) les polyacrylamides comportant des groupements carboxylates,
F) l'acide désoxyribonucléique ;
G) les copolymères d'au moins un diacide carboxylique, d'au moins un diol et d'au moins un monomère aromatique bifonctionnel portant un groupement -S03M avec M représentant un atome d'hydrogène, un ion ammonium NH4+ ou un ion métallique ;
   - et leurs mélanges.

Les polymères anioniques les plus particulièrement préférés sont choisis parmi les polymères anioniques non réticulés comme les copolymères méthylvinyléther / anhydride maléique mono estérifiés commercialisés sous la dénomination GANTREZ ES 425 par la société ISP, les terpolymères acide acrylique / acrylate d'éthyle / N-tertiobutylacrylamide commercialisés sous la dénomination ULTRAHOLD STRONG par la société BASF, les copolymères d'acide méthacrylique et de méthacrylate de méthyle commercialisés sous la dénomination EUDRAGIT L par la société ROHM PHARMA, les terpolymères acétate de vinyle / tertio-butyl benzoate de vinyle / acide crotonique et les terpolymères acide crotonique / acétate de vinyle / néododécanoate de vinyle commercialisés sous la dénomination Résine 28-29-30 par la société NATIONAL STARCH, les copolymères d'acide méthacrylique et d'acrylate d'éthyle commercialisés sous la dénomination LUVIMER MAEX OU MAE par la société BASF, les terpolymères vinylpyrrolidone / acide acrylique / méthacrylate de lauryle commercialisés sous la dénomination ACRYLIDONE LM par la société ISP et les homopolymères d'acide acrylique ou méthacrylique commercialisés par exemple sous la dénomination VERSICOL E 5 OU le polyméthacrylate de sodium vendu sous la dénomination DARVAN 7 par la société VANDERBILT, et leurs mélanges.

De préférence, le polymère anionique est un polyméthacrylate de sodium.

### Epaississant hydrophile

La composition selon l'invention peut comprendre au moins un épaississant hydrophile.

Ces épaississants peuvent être utilisés seuls ou en association. Ces épaississants peuvent être notamment choisis parmi les gommes et les polymères cellulosiques.

Par épaississant hydrophile, on entend un agent épaississant soluble ou dispersible dans l'eau.

Comme épaississants hydrophiles, on peut citer en particulier les polymères épaississants hydrosolubles ou hydrodispersibles. Ceux-ci peuvent notamment être choisis parmi :
- les polymères carboxyvinyliques modifiés ou non, tels que les produits commercialisés sous les dénominations Carbopol (nom CTFA : carbomer) par la société Goodrich ;
- les homo- ou copolymères d'acides acrylique ou méthacrylique ou leurs sels et leurs esters et en particulier les produits vendus sous les dénominations VERSICOL F^{®} ou VERSICOL K^{®} par la société ALLIED COLLOID, UTRAHOLD 8^{®} par la société CIBA-GEIGY, les polyacrylates et polyméthacrylates tels que les produits vendus sous les dénominations de Lubrajel et Norgel par la société GUARDIAN ou sous la dénomination Hispagel par la société HISPANO CHIMICA, les acides polyacryliques de type SYNTHALEN K ;
- les polyacrylamides ;
- les copolymères d'acide acrylique et d'acrylamide vendus sous la forme de leur sel de sodium sous les dénominations RETEN^{®} par la société HERCULES, le polyméthacrylate de sodium vendu sous la dénomination DARVAN N°7^{®} par la société VANDERBILT, les sels de sodium d'acides polyhydroxycarboxyliques vendus sous la dénomination HYDAGEN F^{®} par la société HENKEL ;
- les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société CLARIANT sous la dénomination « Hostacerin AMPS » (nom CTFA : ammonium polyacryldimethyltauramide) ;
- les copolymères anioniques réticulés d'acrylamide et d'AMPS, se présentant sous la forme d'une émulsion E/H, tels ceux commercialisés sous le nom de SEPIGEL 305 (nom C.T.F.A. : Polyacrylamide / C13-14 Isoparaffin / Laureth-7) et sous le nom de SIMULGEL 600 (nom C.T.F.A. : Acrylamide / Sodium acryloyldimethyltaurate copolymer / Isohexadecane / Polysorbate 80) par la société SEPPIC ;
- les copolymères acide polyacryliques/acrylates d'alkyle de type PEMULEN ;
- les polymères associatifs comme le PEG-150/STEARYL ALCOHOL/SMDI COPOLYMER vendu sous le nom Aculyn 46 par Rohm & Haas, ou le STEARETH-100/PEG-136/HDI COPOLYMER vendu sous le nom Rhéolate FX 1100 par Elementis) ;
- et leurs mélanges.

L'épaississant hydrophile peut être choisi parmi les polymères associatifs. Par "polymère associatif' au sens de la présente invention, on entend tout polymère amphiphile comportant dans sa structure au moins une chaîne grasse et au moins une portion hydrophile. Les polymères associatifs conformes à la présente invention peuvent être anioniques, cationiques, non-ioniques ou amphotères.

Parmi les polymères anioniques associatifs, on peut citer ceux comportant au moins un motif hydrophile, et au moins un motif éther d'allyle à chaîne grasse, plus particulièrement parmi ceux dont le motif hydrophile est constitué par un monomère anionique insaturé éthylénique, plus particulièrement par un acide carboxylique vinylique et tout particulièrement par un acide acrylique, un acide méthacrylique ou leurs mélanges, et dont le motif éther d'allyle à chaîne grasse correspond au monomère de formule (I) suivante :

CH₂ = C(R')CH₂ O Bₙ R (I)

dans laquelle R' désigne H ou CH₃, B désigne le radical éthylèneoxy, n est nul ou désigne un entier allant de 1 à 100, R désigne un radical hydrocarboné choisi parmi les radicaux alkyl, arylalkyle, aryle, alkylaryle, cycloalkyle, comprenant 8 à 30 atomes de carbone, de préférence 10 à 24, et plus particulièrement encore de 12 à 18 atomes de carbone.

Des polymères amphiphiles anioniques de ce type sont décrits et préparés, selon un procédé de polymérisation en émulsion, dans le brevet EP-0 216 479.

Comme polymères anioniques associatifs, on peut citer également les polymères anioniques comportant au moins un motif hydrophile de type acide carboxylique insaturé oléfinique, et au moins un motif hydrophobe exclusivement de type ester d'alkyl (C₁₀-C₃₀) d'acide carboxylique insaturé. On peut citer à titre d'exemple les polymères anioniques décrits et préparés, selon les brevets US-3 915 921 et 4 509 949.

Comme polymères associatifs cationiques, on peut citer les dérivés de cellulose quaternisée et les polyacrylates à groupements latéraux aminés.

Les polymères associatifs non-ioniques peuvent être choisis parmi :
- les celluloses modifiées par des groupements comportant au moins une chaîne grasse comme par exemple les hydroxyéthylcelluloses modifiées par des groupements comportant au moins une chaîne grasse tels que des groupes alkyle, notamment en C₈-C₂₂ , arylalkyle, alkylaryle, telles que le NATROSOL PLUS GRADE 330 CS (alkyles en C₁₆) vendu par la société AQUALON,
- les celluloses modifiées par des groupes polyalkylène glycol éther d'alkyl phénol,
- les guars tels que l'hydroxypropyl guar, modifiés par des groupements comportant au moins une chaîne grasse telle qu'une chaîne alkyle,
- les copolymères de vinyl pyrrolidone et de monomères hydrophobes à chaîne grasse ;
- les copolymères de méthacrylates ou d'acrylates d'alkyles en C₁-C₆ et de monomères amphiphiles comportant au moins une chaîne grasse,
- les copolymères de méthacrylates ou d'acrylates hydrophiles et de monomères hydrophobes comportant au moins une chaîne grasse tels que par exemple le copolymère méthacrylate de polyéthylèneglycol/méthacrylate de lauryle,
- les polyuréthanes associatifs

Les polyuréthanes associatifs sont des copolymères séquencés non ioniques comportant dans la chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.

En particulier, ces polymères comportent au moins deux chaînes lipophiles hydrocarbonées, ayant de C₆ à C₃₀ atomes de carbone, séparées par une séquence hydrophile, les chaînes hydrocarbonées peuvent être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile. Les polyuréthanes associatifs peuvent être séquencés sous forme de tribloc ou multibloc. Les séquences hydrophobes peuvent donc être à chaque extrémité de la chaîne (par exemple : copolymère tribloc à séquence centrale hydrophile) ou réparties à la fois aux extrémités et dans la chaîne (copolymère multiséquencé par exemple). Ces polymères peuvent être également en greffons ou en étoile. De préférence, les polyuréthanes associatifs sont des copolymères triblocs dont la séquence hydrophile est une chaîne polyoxyéthylénée comportant de 50 à 1 000 groupements oxyéthylénés. En général les polyuréthannes associatifs comportent une liaison uréthane entre les séquences hydrophiles, d'où l'origine du nom.

A titre d'exemple, des polymères associatifs utilisables dans l'invention, on peut citer le polymère C₁₆-OE₁₂₀-C₁₆ de la société SERVO DELDEN (sous le nom SER AD FX1100, molécule à fonction uréthanne et poids moléculaire moyen en poids de 1300), OE étant un motif oxyéthyléné. Comme polymère associatif, on peut aussi utiliser aussi le Rhéolate 205 à fonction urée vendu par la société RHEOX ou encore le Rhéolate 208 ou 204 ou encore le Rhéolate FX 1100 par Elementis. Ces polyuréthannes associatifs sont vendus sous forme pure. Le produit DW 1206B de chez RHOM & HAAS à chaîne alkyle en C₂₀ et à liaison uréthane, vendu à 20 % en matière sèche dans l'eau, peut aussi être utilisé.

On peut aussi utiliser des solutions ou dispersions de ces polymères notamment dans l'eau ou en milieu hydroalcoolique. A titre d'exemple, de tels polymères on peut citer, le SER AD FX1010, le SER AD FX1035 et le SER AD 1070 de la société SERVO DELDEN, le Rhéolate 255, le Rhéolate 278 et le Rhéolate 244 vendus par la société RHEOX. On peut aussi utiliser le produit Aculyn 46, DW 1206F et le DW 1206J, ainsi que l'Acrysol RM 184 ou l'Acrysol 44 de la société ROHM & HAAS, ou bien encore le Borchigel LW 44 de la société BORCHERS,
et leurs mélanges.

Selon un mode de réalisation avantageux, le polymère épaississant hydrophile utilisé dans la composition de l'invention est choisi parmi les copolymères issus de la polymérisation d'au moins un monomère (a) choisi parmi les acides carboxyliques à insaturation α,β-éthylénique ou leurs esters, avec au moins un monomère (b) à insaturation éthylénique comportant un groupement hydrophobe.

Ce polymère peut aussi présenter des propriétés émulsionnantes.

Le polymère épaississant est de préférence anionique.

On entend par « copolymères » aussi bien les copolymères obtenus à partir de deux sortes de monomères que ceux obtenus à partir de plus de deux sortes de monomères tels que les terpolymères obtenus à partir de trois sortes de monomères.

Leur structure chimique comprend plus particulièrement au moins un motif hydrophile et au moins un motif hydrophobe. Par groupement ou motif hydrophobe, on entend un radical à chaîne hydrocarbonée, saturée ou non, linéaire ou ramifiée, comprenant au moins 8 atomes de carbone, de préférence de 10 à 30 atomes de carbone, en particulier de 12 à 30 atomes de carbone et plus préférentiellement de 18 à 30 atomes de carbone.

De préférence, les copolymères épaississants sont choisis parmi les copolymères issus de la polymérisation :
- d'au moins un monomère de formule (1) suivante : 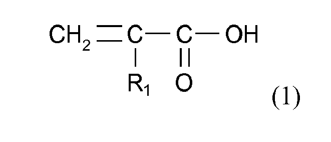 dans laquelle, R₁ désigne H ou CH₃ ou C₂H₅, c'est-à-dire des monomères acide acrylique, acide méthacrylique ou acide éthacrylique, et
- d'au moins un monomère de type ester d'alkyl (C₁₀-C₃₀) d'acide carboxylique insaturé correspondant au monomère de formule (2) suivante : 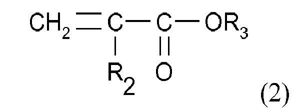 dans laquelle, R₂ désigne H ou CH₃ ou C₂H₅ (c'est-à-dire des motifs acrylates, méthacrylates ou éthacrylates) et de préférence H (motifs acrylates) ou CH₃ (motifs méthacrylates), R₃ désignant un radical alkyle en C₁₀-C₃₀, et de préférence en C₁₂-C₂₂.

Les esters d'alkyles (C₁₀-C₃₀) d'acides carboxyliques insaturés sont choisis de préférence parmi l'acrylate de lauryle, l'acrylate de stéaryle, l'acrylate de décyle, l'acrylate d'isodécyle, l'acrylate de dodécyle, et les méthacrylates correspondants, tels que le méthacrylate de lauryle, le méthacrylate de stéaryle, le méthacrylate de décyle, le méthacrylate d'isodécyle, et le méthacrylate de dodécyle et leurs mélanges.

Selon un mode de réalisation préféré, ces polymères épaississants sont réticulés.

Parmi ce type de copolymères épaississants, on utilisera plus particulièrement des polymères issus de la polymérisation d'un mélange de monomères comprenant :
(i) essentiellement de l'acide acrylique,
(ii) un ester de formule (2) décrite ci-dessus et dans laquelle R₂ désigne H ou CH₃, R₃ désignant un radical alkyle ayant de 12 à 22 atomes de carbone,
(iii) et un agent réticulant, qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.

Parmi ce type de copolymères, on utilisera plus particulièrement ceux constitués de 95 à 60% en poids d'acide acrylique (motif hydrophile), 4 à 40% en poids d'acrylate d'alkyles en C₁₀-C₃₀ (motif hydrophobe), et 0 à 6% en poids de monomère polymérisable réticulant, ou bien ceux constitués de 98 à 96% en poids d'acide acrylique (motif hydrophile), 1 à 4% en poids d'acrylate d'alkyles en C₁₀-C_{30 (}motif hydrophobe), et 0,1 à 0,6% en poids de monomère polymérisable réticulant tel que ceux décrits précédemment.

Parmi lesdits polymères ci-dessus, on préfère tout particulièrement selon la présente invention, les copolymères acrylate/C₁₀-C₃₀-alkylacrylate (nom INCI Acrylates/C10-30 Alkyl acrylate Crosspolymer) tels que les produits commercialisés par la société Lubrizol sous les dénominations commerciales PEMULEN TR1, PEMULEN TR2, CARBOPOL 1382, CARBOPOL EDT 2020 et encore plus préférentiellement le PEMULEN TR-2 .

Le polymère épaississant hydrophile peut représenter, en matière active, de 0,01 à 10% en poids, de préférence de 0,05 à 5 % en poids, mieux de 0,05 à 3 % en poids, encore mieux de 0,05 à 1 % en poids par rapport au poids total de la composition.

### PHASE GRASSE

La composition selon l'invention peut également comprendre une phase grasse comprenant au moins une huile et/ou au moins une cire, et/ou au moins un polymère filmogène lipophile et/ou un agent rhéologique de phase grasse.

### Huiles

Par « huile » on entend un corps gras liquide à température ambiante et pression atmosphérique.

### Huile volatile

La composition selon l'invention peut comprendre au moins une huile volatile.

Par "huile volatile", on entend une huile (ou milieu non aqueux) susceptible de s'évaporer au contact de la peau en moins d'une heure, à température ambiante et pression atmosphérique. L'huile volatile est une huile cosmétique volatile, liquide à température ambiante, ayant notamment une pression de vapeur non nulle, à température ambiante et pression atmosphérique, en particulier ayant une pression de vapeur allant de 0,13 Pa à 40 000 Pa (10-3 à 300 mm de Hg), et de préférence allant de 1,3 Pa à 13 000 Pa (0,01 à 100 mm de Hg), et préférentiellement allant de 1,3 Pa à 1300 Pa (0,01 à 10 mm de Hg).

Cette huile volatile peut être hydrocarbonée.

L'huile volatile hydrocarbonée peut être choisie parmi les huiles hydrocarbonées ayant de 7 à 16 atomes de carbone. L'huile volatile hydrocarbonée peut être présente dans la composition selon l'invention en une teneur allant de 0,1 % à 90% en poids, par rapport au poids total de la composition, de préférence allant de 1% à 70% en poids, et préférentiellement allant de 5% à 70% en poids, voire de 5% à 50% en poids.

La composition selon l'invention peut contenir un ou plusieurs alcane(s) ramifié(s) volatil(s). Par « un ou plusieurs alcane(s) ramifié(s) volatil(s) », on entend indifféremment « une ou plusieurs huile alcane(s) ramifié(s) volatile(s) ».

Comme huile volatile hydrocarbonée ayant de 7 à 16 atomes de carbone, on peut citer notamment les alcanes ramifiés en C8-C16 comme les iso-alcanes (appelées aussi isoparaffines) en C8-C16, l'isododécane, l'isodécane, l'isohexadécane et par exemple les huiles vendues sous les noms commerciaux d'Isopars ou de Permetyls, les esters ramifiés en C8-C16 comme le néopentanoate d'iso-hexyle, et leurs mélanges. De préférence, l'huile volatile hydrocarbonée ayant de 8 à 16 atomes de carbone est choisie parmi l'isododécane, l'isodécane, l'isohexadécane et leurs mélanges, et est notamment l'isododécane.

La composition selon l'invention peut contenir un ou plusieurs alcane(s) linéaire(s) volatil(s). Par « un ou plusieurs alcane(s) linéaire(s) volatil(s) », on entend indifféremment « une ou plusieurs huile alcane(s) linéaire(s) volatile(s) ».

Un alcane linéaire volatil convenant à l'invention est liquide à température ambiante (environ 25 °C) et à la pression atmosphérique (760 mm Hg).

Par « alcane linéaire volatil » convenant à l'invention, on entend un alcane linéaire cosmétique, susceptible de s'évaporer au contact de la peau en moins d'une heure, à température ambiante (25°C) et pression atmosphérique (760 mm Hg, c'est-à-dire 101 325 Pa), liquide à température ambiante, ayant notamment une vitesse d'évaporation allant de 0,01 à 15 mg/cm²/min, à température ambiante (25°C) et pression atmosphérique (760 mm Hg).

De façon préférée, les « alcanes linéaires volatils » convenant à l'invention présentent une vitesse d'évaporation allant de 0,01 à 3,5 mg/cm²/min, à température ambiante (25°C) et pression atmosphérique (760 mm Hg).

De façon préférée, les « alcanes linéaires volatils » convenant à l'invention présentent une vitesse d'évaporation allant de 0,01 à 1,5 mg/cm²/min, à température ambiante (25°C) et pression atmosphérique (760 mm Hg).

De façon plus préférée, les « alcanes linéaires volatils » convenant à l'invention présentent une vitesse d'évaporation allant de 0,01 à 0,8 mg/cm²/min, à température ambiante (25°C) et pression atmosphérique (760 mm Hg).

De façon encore préférée, les « alcanes linéaires volatils » convenant à l'invention présentent une vitesse d'évaporation allant de 0,01 à 0,3 mg/cm²/min, à température ambiante (25°C) et pression atmosphérique (760 mm Hg).

De façon encore préférée, les « alcanes linéaires volatils » convenant à l'invention présentent une vitesse d'évaporation allant de 0,01 à 0,12 mg/cm²/min, à température ambiante (25°C) et pression atmosphérique (760 mm Hg).

La vitesse d'évaporation d'un alcane volatil conforme à l'invention (et plus généralement d'un solvant volatil) peut être notamment évaluée au moyen du protocole décrit dans WO 06/013413, et plus particulièrement au moyen du protocole décrit ci-après.

On introduit dans un cristallisoir (diamètre : 7 cm) placé sur une balance se trouvant dans une enceinte d'environ 0,3 m³ régulée en température (25 °C) et en hygrométrie (humidité relative 50 %) 15 g de solvant hydrocarboné volatil.

On laisse le liquide s'évaporer librement, sans l'agiter, en assurant une ventilation par un ventilateur (PAPST-MOTOREN, référence 8550 N, tournant à 2700 tours/minute) disposé en position verticale au-dessus du cristallisoir contenant le solvant hydrocarboné volatil, les pales étant dirigées vers le cristallisoir, à une distance de 20 cm par rapport au fond du cristallisoir.

On mesure à intervalles de temps réguliers la masse de solvant hydrocarboné volatil restante dans le cristallisoir.

On obtient alors le profil d'évaporation du solvant en traçant la courbe de la quantité de produit évaporé (en mg/cm²) en fonction du temps (en min).

Puis on calcule la vitesse d'évaporation qui correspond à la tangente à l'origine de la courbe obtenue. Les vitesses d'évaporation sont exprimées en mg de solvant volatil évaporé par unité de surface (cm²) et par unité de temps (minute).

Selon un mode de réalisation préféré, les « alcanes linéaires volatils » convenant à l'invention ont une pression de vapeur (appelée également pression de vapeur saturante) non nulle, à température ambiante, en particulier une pression de vapeur allant de 0,3 Pa à 6000 Pa.

De façon préférée, les « alcanes linéaires volatils » convenant à l'invention ont une pression de vapeur allant de 0,3 à 2000 Pa, à température ambiante (25°C).

De façon préférée, les « alcanes linéaires volatils » convenant à l'invention ont une pression de vapeur allant de 0,3 à 1000 Pa, à température ambiante (25°C)

De façon plus préférée, les « alcanes linéaires volatils » convenant à l'invention ont une pression de vapeur allant de 0,4 à 600 Pa, à température ambiante (25°C).

De façon préférée, les « alcanes linéaires volatils » convenant à l'invention ont une pression de vapeur allant de 1 à 200 Pa, à température ambiante (25°C).

De façon encore préférée, les « alcanes linéaires volatils » convenant à l'invention ont une pression de vapeur allant de 3 à 60 Pa, à température ambiante (25°C).

Selon un mode de réalisation, un alcane linéaire volatil convenant à l'invention peut présenter un point éclair compris dans l'intervalle variant de 30 à 120 °C, et plus particulièrement de 40 à 100 °C. Le point éclair est en particulier mesuré selon la Norme iso 3679.

Selon un mode de réalisation, un alcane convenant à l'invention peut être un alcane linéaire volatil comprenant de 7 à 14 atomes de carbone.

De façon préférée, les « alcanes linéaires volatils » convenant à l'invention comprennent de 8 à 14 atomes de carbone.

De façon préférée, les « alcanes linéaires volatils » convenant à l'invention comprennent de 9 à 14 atomes de carbone.

De façon préférée, les « alcanes linéaires volatils » convenant à l'invention comprennent de 10 à 14 atomes de carbone.

De façon préférée, les « alcanes linéaires volatils » convenant à l'invention comprennent de 11 à 14 atomes de carbone.

Selon un mode de réalisation avantageux, les « alcanes linéaires volatils » convenant à l'invention présentent une vitesse d'évaporation, telle que définie plus haut, allant de 0,01 à 3,5 mg/cm²/min, à température ambiante (25°C) et pression atmosphérique (760 mm Hg), et comprennent de 8 à 14 atomes de carbone.

Un alcane linéaire volatil convenant à l'invention peut être avantageusement d'origine végétale.

De préférence, l'alcane linéaire volatil ou le mélange d'alcanes linéaires volatils présent dans la composition selon l'invention comprend au moins un isotope ¹⁴C du carbone (carbone 14), en particulier l'isotope ¹⁴C peut être présent en un ratio ¹⁴C / ¹²C supérieur ou égal à 1.10⁻¹⁶, de préférence supérieur ou égal à 1.10⁻¹⁵ , de préférence encore supérieur ou égal 7,5.10⁻¹⁴, et mieux supérieur ou égal 1,5.10⁻¹³. De préférence, le ratio ¹⁴C /¹²C va de 6.10⁻¹³ à 1,2.10⁻¹².

La quantité de d'isotopes ¹⁴C dans l'alcane linéaire volatil ou le mélange d'alcanes linéaires volatils peut être déterminée par des méthodes connues de l'homme du métier telles que la méthode de comptage de Libby, la spectrométrie à scintillation liquide ou encore la spectrométrie de masse à accélération (Accelerator Mass Spectrometry).

Un tel alcane peut être obtenu, directement ou en plusieurs étapes, à partir d'une matière première végétale comme une huile, un beurre, une cire, etc.

A titre d'exemple d'alcanes convenant à l'invention, on peut mentionner les alcanes décrits dans les demandes de brevets de la société Cognis WO 2007/068371, ou WO2008/155059 (mélanges d'alcanes distincts et différant d'au moins un carbone). Ces alcanes sont obtenus à partir d'alcools gras, eux-mêmes obtenus à partir d'huile de coprah ou de palme.

A titre d'exemple d'alcanes linéaires convenant à l'invention, on peut citer le n- heptane (C7), le n-octane (C8), le n-nonane (C9), le n-décane (C10), le n-undécane (C11), le n-dodécane (C12), le n-tridécane (C13), le n-tétradecane (C14), et leurs mélanges. Selon un mode de réalisation particulier, l'alcane linéaire volatil est choisi parmi le n-nonane, le n-undécane, le n-dodécane, le n-tridécane, le n-tétradécane, et leurs mélanges.

Selon un mode préféré, on peut citer les mélanges de n-undécane (C11) et de n-tridécane (C13) obtenus aux exemples 1 et 2 de la demande WO2008/155059 de la Société Cognis.

On peut également citer le n-dodécane (C12) et le n-tétradécane (C14) vendus par Sasol respectivement sous les références PARAFOL 12-97 et PARAFOL 14-97, ainsi que leurs mélanges.

On pourra utiliser l'alcane linéaire volatil seul.

On pourra alternativement ou préférentiellement utiliser un mélange d'au moins deux alcanes linéaires volatils distincts, différant entre eux d'un nombre de carbone n d'au moins 1, en particulier différant entre eux d'un nombre de carbone de 1 ou de 2.

Selon un premier mode de réalisation, on utilise un mélange d'au moins deux alcanes linéaires volatils distincts comportant de 10 à 14 atomes de carbone et différant entre eux d'un nombre de carbone d'au moins 1. A titre d'exemples, on peut citer notamment les mélanges d'alcanes linéaires volatils C10/C11, C11/C12, ou C12/C13.

Selon un autre mode de réalisation, on utilise un mélange d'au moins deux alcanes linéaires volatils distincts comportant de 10 à 14 atomes de carbone et différant entre eux d'un nombre de carbone d'au moins 2. A titre d'exemples, on peut citer notamment les mélanges d'alcanes linéaires volatils C10/C12, ou C12/C14, pour un nombre de carbone n pair et le mélange C11/C13 pour un nombre de carbone n impair.

Selon un mode préféré, on utilise un mélange d'au moins deux alcanes linéaires volatils comportant de 10 à 14 atomes de carbone distincts et différant entre eux d'un nombre de carbone d'au moins 2, et en particulier un mélange d'alcanes linéaires volatils C11/C13 ou un mélange d'alcanes linéaires volatils C12/C14.

D'autres mélanges associant plus de 2 alcanes linéaires volatils selon l'invention, tels que par exemple un mélange d'au moins 3 alcanes linéaires volatils comportant de 7 à 14 atomes de carbone distincts et différant entre eux d'un nombre de carbone d'au moins 1, font également partie de l'invention, mais les mélanges de 2 alcanes linéaires volatils selon l'invention sont préférés (mélanges binaires), lesdits 2 alcanes linéaires volatils représentant de préférence plus de 95% et mieux plus de 99% en poids de la teneur totale en alcanes linéaires volatils dans le mélange. Selon un mode particulier de l'invention, dans un mélange d'alcanes linéaires volatils, l'alcane linéaire volatil ayant le nombre de carbone le plus petit est majoritaire dans le mélange.

Selon un autre mode de l'invention, on utilise un mélange d'alcanes linéaires volatils dans lequel l'alcane linéaire volatil ayant le nombre de carbone le plus grand est majoritaire dans le mélange.

A titre d'exemples de mélanges convenant à l'invention, on peut citer notamment les mélanges suivants :
- de 50 à 90% en poids, de préférence de 55 à 80% en poids, préférentiellement encore de 60 à 75% en poids d'alcane linéaire volatil en Cn avec n allant de 7 à 14
- de 10 à 50% en poids, de préférence de 20 à 45% en poids, de préférence de 24 à 40% en poids, d'alcane linéaire volatil en Cn+x avec x supérieur ou égal à 1, de préférence x=1 ou x=2, avec n+x compris entre 8 et 14,
par rapport au poids total des alcanes dans ledit mélange.

En particulier, ledit mélange d'alcanes selon l'invention contient :
- moins de 2% en poids, de préférence moins de 1% en poids d'hydrocarbures ramifiés,
- et/ou moins de 2% en poids, de préférence moins de 1% en poids d'hydrocarbures aromatiques,
- et/ou moins de 2% en poids, de préférence moins de 1% en poids et préférentiellement moins de 0.1% en poids d'hydrocarbures insaturés dans le mélange.

Plus particulièrement, un alcane linéaire volatil convenant à l'invention peut être mis en oeuvre sous la forme d'un mélange n-undécane/n-tridécane.

En particulier, on utilisera un mélange d'alcanes linéaires volatils comprenant :
- de 55 à 80% en poids, de préférence de 60 à 75% en poids d'alcane linéaire volatil en C11 (n-undécane)
- de 20 à 45% en poids, de préférence de 24 à 40% en poids d'alcane linéaire volatil en C13 (n-tridécane)
par rapport au poids total des alcanes dans ledit mélange.

Selon un mode de réalisation particulier, le mélange d'alcanes est un mélange n-undécane/n-tridécane. En particulier un tel mélange peut être obtenu selon l'exemple 1 ou l'exemple 2 du WO 2008/155059.

Selon un autre mode de réalisation particulier, on utilise le n-dodécane vendu sous la référence PARAFOL 12-97 par SASOL.

Selon un autre mode de réalisation particulier, on utilise le n-tétradécane vendu sous la référence PARAFOL 14-97 par SASOL.

Selon encore un autre mode de réalisation, on utilise un mélange de n-dodécane et de n-tétradécane.

En variante ou de façon additionnelle, la composition réalisée peut comprendre au moins une huile ou solvant siliconée volatile, compatible avec une utilisation cosmétique.

Par huile siliconée, on entend une huile contenant au moins un atome de silicium, et notamment contenant des groupes Si-O. Selon un mode de réalisation, ladite composition comprend moins de 10% en poids d'huile(s) siliconée(s) non volatiles, par rapport au poids total de la composition, mieux moins de 5% en poids, voire est exempte d'huile siliconée.

Comme huile volatile siliconée, on peut citer les polysiloxanes cycliques, les polysiloxanes linéaires et leurs mélanges. Comme polysiloxanes volatiles linéaires, on peut citer l'hexamethyldisiloxane, l'octamethyltrisiloxane, le decamethyltetrasiloxane, le tetradecamethylhexasiloxane et l'hexadecamethylheptasiloxane. Comme polysiloxanes volatiles cycliques, on peut citer l'hexamethylcyclotrisiloxane, l'octamethylcylotetrasiloxane, le decamethylcyclopentasiloxane et le dodecamethylcyclohexasiloxane.

En variante ou de façon additionnelle, la composition réalisée peut comprendre au moins une huile volatile fluorée.

Par huile fluorée, on entend une huile contenant au moins un atome de fluor.

Comme huile volatile fluorée, on peut citer le nonafluorométhoxybutane ou le perfluorométhylcyclopentane, et leurs mélanges.

L'huile volatile peut être présente en une teneur allant de 0.1 % à 90 % en poids, par rapport au poids total de la composition, de préférence allant de 1 % à 70 % en poids, et préférentiellement allant de 5 % à 50 % en poids.

### Huile non volatile

La composition selon l'invention peut comprendre au moins une huile non volatile.

L'huile peut être choisie parmi les huiles hydrocarbonées, les huiles siliconées, les huiles fluorées.

Comme huile non volatile hydrocarbonée, on peut utiliser l'huile de paraffine ( ou vaseline), le squalane, le polyisobutylène hydrogéné (huile de Parléam), le perhydrosqualène, l'huile de vison, de tortue, de soja, l'huile d'amande douce, de calophyllum, de palme, de pépins de raisin, de sésame, de maïs, d'arara, de colza, de tournesol, de coton, d'abricot, de ricin, d'avocat, de jojoba, d'olive ou de germes de céréales ; des esters d'acide lanolique, d'acide oléique, d'acide laurique, d'acide stéarique ; les esters gras, notamment en C12-C36, tels que le myristate d'isopropyle, le palmitate d'isopropyle, le stéarate de butyle, le laurate d'hexyle, l'adipate de diisopropyle, l'isononanoate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de di(2-éthyl hexyle), le malate de diisostéaryle, le triisostéarate de glycérine ou de diglycérine ; l'acide béhénique, l'acide oléique, l'acide linoléique, l'acide linolénique ou l'acide isostéarique ; les alcools gras supérieurs, notamment en C16- C22, tels que le cétanol, l'alcool oléique, l'alcool linoléique ou linolénique, l'alcool isostéarique ou l'octyl dodécanol ; et leurs mélanges.

Les huiles fluorées utilisables dans l'invention sont notamment des huiles fluorosiliconées, des polyéthers fluorés, des silicones fluorées telles que décrit dans le document EP-A-847752.

L'huile non volatile peut être présente en une teneur allant de 0.1 % à 70 % en poids, par rapport au poids total de la composition, de préférence allant de 0.5 % à 60 % en poids, et préférentiellement allant de 1 % à 50 % en poids.

### Cires

La composition peut comprendre au moins une cire.

Par cire au sens de la présente invention, on entend un composé lipophile, solide à température ambiante (25 °C), à changement d'état solide/liquide réversible, ayant un point de fusion supérieur ou égal à 30 °C pouvant aller jusqu'à 120 °C.

Le point de fusion de la cire peut être mesuré à l'aide d'un calorimètre à balayage différentiel (D.S.C.), par exemple le calorimètre vendu sous la dénomination DSC 30 par la société METLER.

Les cires peuvent être hydrocarbonées, fluorées et/ou siliconées et être d'origine végétale, minérale, animale et/ou synthétique. En particulier, les cires présentent une température de fusion supérieure à 25 °C et mieux supérieure à 45 °C.

La cire peut être présente en une teneur au moins égale à 15% en poids. De préférence, elle est présente en une teneur allant de 15 à 40% en poids par rapport au poids total de la composition, mieux de 16 à 35% et encire mieux de 16 à 30% en poids. La cire peut être présente dans la composition en une teneur allant de 0,1 % à 50 % en poids, par rapport au poids total de la composition, de préférence allant de 1 % à 40 % en poids, et préférentiellement allant de 5% à 30% en poids.

On peut notamment utiliser les cires hydrocarbonées comme la cire d'abeilles, la cire de lanoline, et les cires d'insectes de Chine; la cire de riz, la cire de Carnauba, la cire de Candellila, la cire d'Ouricurry, la cire d'Alfa, la cire de fibres de liège, la cire de canne à sucre, la cire du Japon et la cire de sumac; la cire de montan, les cires microcristallines, les paraffines et l'ozokérite; les cires de polyéthylène, les cires obtenues par la synthèse de Fisher-Tropsch et les copolymères cireux ainsi que leurs esters.

On peut aussi citer les cires obtenues par hydrogénation catalytique d'huiles animales ou végétales ayant des chaînes grasses, linéaires ou ramifiées, en C8-C32.

Parmi celles-ci, on peut notamment citer l'huile de jojoba hydrogénée, l'huile de jojoba isomérisée telle que l'huile de jojoba partiellement hydrogénée isomérisée trans fabriquée ou commercialisée par la société Désert Whale sous la référence commerciale ISO-JOJOBA-50®, l'huile de tournesol hydrogénée, l'huile de ricin hydrogénée, l'huile de coprah hydrogénée et l'huile de lanoline hydrogénée, le tétrastéarate de di-(triméthylol-1,1,1 propane) vendu sous la dénomination "HEST 2T-4S" par la société HETERENE, le tétrabéhénate de di-(triméthylol-1,1,1 propane) vendue sous la dénomination HEST 2T-4B par la société HETERENE.

On peut encore citer les cires de silicone comme les alkyl ou alkoxy-diméticone ayant de 16 à 45 atomes de carbone, les cires fluorées.

On peut également utiliser la cire obtenue par hydrogénation d'huile d'olive estérifiée avec l'alcool stéarylique vendue sous la dénomination "PHYTOWAX Olive 18 L 57" ou bien encore les cires obtenues par hydrogénation d'huile de ricin estérifiée avec l'alcool cétylique vendus sous la dénomination "PHYTOWAX ricin 16L64 et 22L73", par la société SOPHIM. De telles cires sont décrites dans la demande FR-A- 2792190.

La composition peut comprendre au moins une cire polaire. Par « cire polaire », on entend des cires comprenant dans leur structure chimique, en plus des atomes de carbone et d'hydrogène, au moins un hétéroatome fortement électronégatif, tels que O, N ou P.

De préférence, la cire est choisie parmi la cire de carnauba, la cire de candellila, la cire d'abeille naturelle (ou blanchie) et la cire d'abeille synthétique. Comme cire d'abeille synthétique, on peut citer la cire vendue sous le nom Cyclochem 326 A par Evonik Goldschmidt (nom INCI : Synthetic Beeswax).

La composition peut comprendre au moins une cire présentant une dureté allant de 0,05 MPa à 15 MPa, et de préférence allant de 6 MPa à 15 MPa. La dureté est déterminée par la mesure de la force en compression mesurée à 20 °C à l'aide du texturomètre vendu sous la dénomination TA-TX2i par la société RHEO, équipé d'un cylindre en inox d'un diamètre de 2 mm se déplaçant à la vitesse de mesure de 0,1 mm/s, et pénétrant dans la cire à une profondeur de pénétration de 0,3 mm.

Selon un mode de réalisation particulier, les compositions conformes à l'invention peuvent comprendre au moins une cire dite cire collante c'est-à-dire possédant un collant supérieur ou égal à 0,7 N.s et une dureté inférieure ou égale à 3,5 MPa.

L'utilisation d'une cire collante peut notamment permettre l'obtention d'une composition cosmétique qui s'applique facilement sur les cils, ayant une bonne accroche sur les cils et qui conduit à la formation d'un maquillage lisse, homogène et épaississant.

La cire collante utilisée peut posséder notamment un collant allant de 0,7 N.s à 30 N.s, en particulier supérieur ou égal à 1 N.s, notamment allant de 1 N.s à 20 N.s, en particulier supérieur ou égal à 2 N.s, notamment allant de 2 N.s à 10 N.s, et en particulier allant de 2 N.s à 5 N.s.

Le collant de la cire est déterminé par la mesure de l'évolution de la force (force de compression ou force d'étirement) en fonction du temps, à 20 °C à l'aide du texturomètre vendu sous la dénomination "TA-TX2i®" par la société RHEO, équipé d'un mobile en polymère acrylique en forme de cône formant un angle de 45°.

Le protocole de mesure est le suivant :

La cire est fondue à une température égale au point de fusion de la cire + 10 °C. La cire fondue est coulée dans un récipient de 25 mm de diamètre et de 20 mm de profondeur. La cire est recristallisée à température ambiante (25 °C) pendant 24 heures de telle sorte que la surface de la cire soit plane et lisse, puis la cire est conservée pendant au moins 1 heure à 20 °C avant d'effectuer la mesure du collant.

Le mobile du texturomètre est déplacé à la vitesse de 0,5 mm/s, puis pénètre dans la cire jusqu'à une profondeur de pénétration de 2 mm. Lorsque le mobile a pénétré dans la cire à la profondeur de 2 mm, le mobile est maintenu fixe pendant 1 seconde (correspondant au temps de relaxation) puis est retiré à la vitesse de 0,5 mm/s.

Pendant le temps de relaxation, la force (force de compression) décroît fortement jusqu'à devenir nulle puis, lors du retrait du mobile, la force (force d'étirement) devient négative pour ensuite croître à nouveau vers la valeur 0. Le collant correspond à l'intégrale de la courbe de la force en fonction du temps pour la partie de la courbe correspondant aux valeurs négatives de la force (force d'étirement). La valeur du collant est exprimée en N.s.

La cire collante pouvant être utilisée a généralement une dureté inférieure ou égale à 3,5 MPa, en particulier allant de 0,01 MPa à 3,5 MPa, notamment allant de 0,05 MPa à 3 MPa, voire encore allant de 0,1 MPa à 2,5 MPa.

La dureté est mesurée selon le protocole décrit précédemment.

Comme cire collante, on peut utiliser un (hydroxystéaryloxy)stéarate d'alkyle en C20-C40 (le groupe alkyle comprenant de 20 à 40 atomes de carbone), seul ou en mélange, en particulier un 12-(12'-hydroxystéaryloxy)stéarate d'alkyle en C20-C40, de formule (II) : 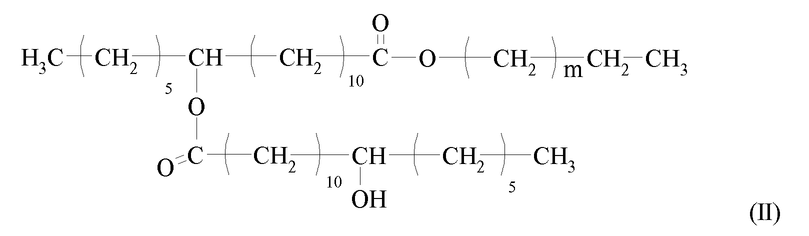 dans laquelle m est un entier allant de 18 à 38, ou un mélange de composés de formule (II).

Une telle cire est notamment vendue sous les dénominations "Kester Wax K 82 P®" et "Kester Wax K 80 P®" par la société KOSTER KEUNEN.

Les cires citées ci-dessus présentent généralement un point de fusion commençante inférieur à 45 °C.

On peut également utiliser la cire microcristalline commercialisée sous la référence SP18 par la société STRAHL and PITSCH qui présente une dureté d'environ 0,46 MPa et une valeur de collant d'environ 1 N.s.

La ou les cires peu(ven)t être présente(s) sous forme d'une microdispersion aqueuse de cire. On entend par microdispersion aqueuse de cire, une dispersion aqueuse de particules de cire, dans laquelle la taille desdites particules de cire est inférieure ou égale à environ 1 µm.

Les microdispersions de cire sont des dispersions stables de particules colloïdales de cire, et sont notamment décrites dans "Microemulsions Theory and Practice", L.M. Prince Ed., Academic Press (1977) pages 21-32.

En particulier, ces microdispersions de cire peuvent être obtenues par fusion de la cire en présence d'un tensioactif, et éventuellement d'une partie de l'eau, puis addition progressive d'eau chaude avec agitation. On observe la formation intermédiaire d'une émulsion du type eau-dans-huile, suivie d'une inversion de phase avec obtention finale d'une microémulsion du type huile-dans-eau. Au refroidissement, on obtient une microdispersion stable de particules colloïdales solides de cire.

Les microdispersions de cire peuvent également être obtenues par agitation du mélange de cire, de tensioactif et d'eau à l'aide de moyen d'agitation tels que les ultrasons, l'homogénéisateur haute pression, les turbines.

Les particules de la microdispersion de cire ont de préférence des dimensions moyennes inférieures à 1 µm (notamment allant de 0,02 µm à 0,99 µm), de préférence inférieures à 0,5 µm (notamment allant de 0,06 µm à 0,5 µm).

Ces particules sont constituées essentiellement d'une cire ou d'un mélange de cires. Elles peuvent toutefois comprendre en proportion minoritaire des additifs gras huileux et/ou pâteux, un tensioactif et/ou un additif/actif liposoluble usuel.

### Polymère filmogène lipophile

La composition selon l'invention peut comprendre au moins un polymère filmogène lipophile qui peut être liposoluble (c'est-à-dire soluble dans une phase grasse liquide comprenant des huiles ou solvants organiques tels que ceux décrits précédemment) ou être présent dans la composition sous la forme de particules en dispersion dans une phase solvant non aqueuse avec laquelle il est compatible, qui peut être la phase huileuse de la composition selon l'invention.

A titre d'exemple de polymère liposoluble, on peut citer les copolymères d'ester vinylique (le groupe vinylique étant directement relié à l'atome d'oxygène du groupe ester et l'ester vinylique ayant un radical hydrocarboné saturé, linéaire ou ramifié, de 1 à 19 atomes de carbone, lié au carbonyle du groupe ester) et d'au moins un autre monomère qui peut être un ester vinylique (différent de l'ester vinylique déjà présent), une α-oléfine (ayant de 8 à 28 atomes de carbone), un alkylvinyléther (dont le groupe alkyl comporte de 2 à 18 atomes de carbone), ou un ester allylique ou méthallylique (ayant un radical hydrocarboné saturé, linéaire ou ramifié, de 1 à 19 atomes de carbone, lié au carbonyle du groupe ester).

Ces copolymères peuvent être réticulés à l'aide de réticulants qui peuvent être soit du type vinylique, soit du type allylique ou méthallylique, tels que le tétraallyloxyéthane, le divinylbenzène, l'octanedioate de divinyle, le dodécanedioate de divinyle, et l'octadécanedioate de divinyle.

Comme exemples de ces copolymères, on peut citer les copolymères : acétate de vinyle/stéarate d'allyle, l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène-1, acétate de vinyle/dodécène-1, stéarate de vinyle/éthylvinyléther, propionate de vinyle/cétyl vinyle éther, stéarate de vinyle/acétate d'allyle, diméthyl-2, 2 octanoate de vinyle/laurate de vinyle, diméthyl-2, 2 pentanoate d'allyle/laurate de vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyl éther, réticulé avec 0,2 % de tétraallyloxyéthane, acétate de vinyle/stéarate d'allyle, réticulé avec 0,2 % de divinyl benzène, acétate de vinyle/octadécène-1 réticulé avec 0,2 % de divinyl benzène et propionate d'allyle/stéarate d'allyle réticulé avec 0,2 % de divinyl benzène.

Comme polymères filmogènes liposolubles, on peut également citer les copolymères liposolubles, et en particulier ceux résultant de copolymérisation d'esters vinyliques ayant de 9 à 22 atomes de carbone ou d'acrylates ou de méthacrylates d'alkyle, les radicaux alkyles ayant de 10 à 20 atomes de carbone.

De tels copolymères liposolubles peuvent être choisis parmi les copolymères de polystéarate de vinyle, de polystéarate de vinyle réticulé à l'aide de divinylbenzène, de diallyléther ou de phtalate de diallyle, les copolymères de poly(méth)acrylate de stéaryle, de polylaurate de vinyle, de poly(méth)acrylate de lauryle, ces poly(méth)acrylates pouvant être réticulés à l'aide de diméthacrylate de l'éthylène glycol ou de tétraéthylène glycol.

Les copolymères liposolubles définis précédemment sont connus et notamment décrits dans la demande FR-A-2232303 ; ils peuvent avoir un poids moléculaire moyen en poids allant de 2.000 à 500.000 et de préférence de 4.000 à 200.000.

Comme polymères filmogènes liposolubles utilisables dans l'invention, on peut également citer les polyalkylènes et notamment les copolymères d'alcènes en C2-C20, comme le polybutène, les alkylcelluloses avec un radical alkyle linéaire ou ramifié, saturé ou non en C1 à C8 comme l'éthylcellulose et la propylcellulose, les copolymères de la vinylpyrolidone (VP) et notamment les copolymères de la vinylpyrrolidone et d'alcène en C2 à C40 et mieux en C3 à C20. A titre d'exemple de copolymère de VP utilisable dans l'invention, on peut citer le copolymère de VP/acétate vinyle, VP/méthacrylate d'éthyle, la polyvinylpyrolidone (PVP) butylée, VP/méthacrylate d'éthyle/acide méthacrylique, VP/eicosène, VP/hexadécène, VP/triacontène, VP/styrène, VP/acide acrylique/méthacrylate de lauryle.

Le polymère filmogène lipophile peut être choisi parmi le copolymère acétate de vinyle/stéarate d'allyle (vendu notamment sous le nom Mexomère PQ par Chimex), le polylaurate de vinyle (vendu notamment sous le nom Mexomère PP par Chimex), le copolymère vinylpyrrolidone/eicosène (vendu notamment sous le nom Antaron V 220 par ISP) et leurs mélanges.

On peut également citer les résines de silicone, généralement solubles ou gonflables dans les huiles de silicone, qui sont des polymères de polyorganosiloxanes réticulés. La nomenclature des résines de silicone est connue sous le nom de "MDTQ", la résine étant décrite en fonction des différentes unités monomèriques siloxane qu'elle comprend, chacune des lettres "MDTQ" caractérisant un type d'unité.

A titre d'exemples de résines polymethylsilsesquioxanes commercialement disponibles, on peut citer celles qui sont commercialisées par la société Wacker sous la référence Resin MK tels que la Belsil PMS MK, ou par la société SHIN-ETSU sous les références KR-220L.

A titre d'exemples de résines polypropylsilsesquioxanes commercialement disponibles, on peut citer celles qui sont commercialisés sous la référence DC670 par la société Dow Corning.

Comme résines siloxysilicates, on peut citer les résines trimethylsiloxysilicate (TMS) telles que celle commercialisées sous la référence SR1000 par la société General Electric ou sous la référence TMS 803 par la société Wacker. On peut encore citer les résines timéthylsiloxysilicate commercialisées dans un solvant tel que la cyclomethicone, vendues sous la dénomination "KF-7312J" par la société Shin-Etsu, "DC 749", "DC 593" par la société Dow Corning.

On peut aussi citer des copolymères de résines de silicone telles que celles citées ci-dessus avec des polydiméthylsiloxanes, comme les copolymères adhésifs sensibles à la pression commercialisés par la société Dow Corning sous la référence BIO-PSA et décrits dans le document US 5 162 410 ou encore les copolymères siliconés issus de la réaction d'un résine de silicone, telle que celles décrite plus haut, et d'un diorganosiloxane tels que décrits dans le document WO 2004/073626.

Le polymère filmogène lipophile peut également être un polymère vinylique comprenant au moins un motif dérivé de dendrimère carbosiloxane.

Conviennent notamment à l'invention les polymères vinyliques comprenant des motifs dérivés de dendrimère carbosiloxane, tels que, par exemple, ceux décrits dans les documents WO 2006/058793 et EP 1 862 162.

Le polymère vinylique peut posséder notamment un squelette et au moins une chaîne latérale, laquelle comprend une structure de dendrimère carbosiloxane. Le terme « structure de dendrimère carbosiloxane » dans le contexte de la présente invention représente une structure moléculaire possédant des groupes ramifiés ayant des masses moléculaires élevées, ladite structure ayant une régularité élevée dans la direction radiale en partant de la liaison au squelette. De telles structures de dendrimère carbosiloxane sont décrites sous la forme d'un copolymère siloxane-silylalkylène fortement ramifié dans la demande de brevet japonais mise à l'inspection publique Kokai 9-171 154.

Les polymères vinyliques greffés avec au moins un motif dérivé de dendrimère carbosiloxane pouvant convenir tout particulièrement à la présente invention sont les polymères vendus sous les dénominations TIB 4-100, TIB 4-101, TIB 4-120, TIB 4-130, TIB 4-200, FA 4002 ID (TIB 4-202), TIB 4-220, FA 4001 CM (TIB 4-230) par la société Dow Corning.

On peut également utiliser les polyamides siliconés du type polyorganosiloxane tels que ceux décrits dans les documents US-A-5,874,069, US-A-5,919,441, US-A-6,051,216 et US-A-5,981,680.

Ces polymères siliconés peuvent appartenir aux deux familles suivantes :
- des polyorganosiloxanes comportant au moins deux groupes capables d'établir des interactions hydrogène, ces deux groupes étant situés dans la chaîne du polymère, et/ou
- des polyorganosiloxanes comportant au moins deux groupes capables d'établir des interactions hydrogène, ces deux groupes étant situés sur des greffons ou ramifications.

Le polymère filmogène lipophile ou liposoluble peut être également présent dans la composition sous la forme de particules en dispersion dans une phase solvant non aqueuse qui peut être la phase huileuse de la composition. Les techniques de préparation de ces dispersions sont bien connues de l'homme du métier. Par exemple, Les polymères de type NAD (non aqueous dispersion) ou des microgels (par exemple les KSG) peuvent être utilisés, ainsi que les copolymères à base de styrène (Kraton, Regalite).

Comme exemples de dispersions non aqueuses de polymère filmogène lipodispersibles sous forme de dispersions non aqueuses de particules de polymère dans une ou plusieurs huiles de silicone et/ou hydrocarbonées et pouvant être stabilisées en leur surface par au moins un agent stabilisant, notamment un polymère séquencé, greffé ou statistique, on peut citer les dispersions acryliques dans l'isododécane comme le Mexomère PAPⓇ de la société CHIMEX, les dispersions de particules d'un polymère éthylénique greffé, de préférence acrylique, dans une phase grasse liquide, le polymère éthylénique étant avantageusement dispersé en l'absence de stabilisant additionnel en surface des particules telles que décrite notamment dans le document WO 04/055081.

Selon un exemple de mise en oeuvre de l'invention, le polymère filmogène est un polymère éthylénique séquencé linéaire filmogène, qui comprend de préférence au moins une première séquence et au moins une deuxième séquence ayant des températures de transition vitreuse (Tg) différentes, lesdites première et deuxième séquences étant reliées entre elles par une séquence intermédiaire comprenant au moins un monomère constitutif de la première séquence et au moins un monomère constitutif de la deuxième séquence.

Avantageusement, les première et deuxième séquences et du polymère séquencé sont incompatibles l'une avec l'autre.

De tels polymères sont décrits par exemple dans les documents EP 1411069 ou WO04/028488.

A titre d'autres exemples de système filmogène utilisable dans les compositions selon l'invention, on peut citer les systèmes dans lequel le film se forme in-situ au moment de l'application de la composition ou d'un mélange de compositions contenant deux composés siliconés réagissant lorsqu'ils sont mis en contact l'un de l'autre. De tels systèmes sont décrits notamment dans la demande WO 2007/071706. Des systèmes de ce type sont également décrits dans les demandes US2007/142575 ou US 2007/142599.

Le polymère filmogène lipophile peut représenter de 0.1% à 15% en poids par rapport au poids total de la composition, de préférence de 0.5% à 10%.

### Agent rhéologique de phase grasse

La composition selon l'invention peut comprendre un agent rhéologique de phase grasse liquide choisi parmi les composés pâteux, les polymères semi-cristallin, les gélifiants lipophiles et leurs mélanges.

Par « agent rhéologique de phase grasse », on entend un agent apte à établir des interactions physiques, le cas échéant au contact d'un agent réticulant, lorsque l'agent structurant n'est pas réticulé, dans ladite phase grasse au sein de laquelle il est mis en oeuvre. Il présente la capacité de développer des propriétés structurantes - par exemple gélifiantes - et conduit ainsi à des textures d'aspect semi-solide.

L'agent rhéologique de phase grasse peut représenter de 0,1 à 60 % en poids par rapport au poids total de la composition, de préférence de 0,5 à 50 % et de façon encore plus préférée de 1 à 40 % en poids.

### Composés pâteux

Par composé pâteux ou corps gras pâteux, on entend un composé gras lipophile comportant à la température de 23°C une fraction liquide et une fraction solide.

Ledit composé pâteux a de préférence une dureté à 20°C allant de 0,001 à 0,5 MPa, de préférence de 0,002 à 0,4 MPa.

La dureté est mesurée selon une méthode de pénétration d'une sonde dans un échantillon de composé et en particulier à l'aide d'un analyseur de texture (par exemple le TA-XT2i de chez Rhéo) équipé d'un cylindre en inox de 2 mm de diamètre. La mesure de dureté est effectuée à 20°C au centre de 5 échantillons. Le cylindre est introduit dans chaque échantillon à une pré-vitesse de 1mm/s puis à une vitesse de mesure de 0,1 mm/s, la profondeur de pénétration étant de 0,3 mm. La valeur relevée de la dureté est celle du pic maximum.

La fraction liquide du composé pâteux mesurée à 23°C représente de préférence 9 à 97% en poids du composé. Cette fraction liquide à 23°C représente de préférence entre 15 et 85%, de préférence encore entre 40 et 85% en poids. La fraction liquide en poids du composé pâteux à 23°C est égale au rapport de l'enthalpie de fusion consommée à 23°C sur l'enthalpie de fusion du composé pâteux.

L'enthalpie de fusion du composé pâteux est l'enthalpie consommée par le composé pour passer de l'état solide à l'état liquide. Le composé pâteux est dit à l'état solide lorsque l'intégralité de sa masse est sous forme solide cristalline. Le composé pâteux est dit à l'état liquide lorsque l'intégralité de sa masse est sous forme liquide.

L'enthalpie de fusion du composé pâteux est égale à l'aire sous la courbe du thermogramme obtenu à l'aide d'un calorimètre à balayage différentiel (D. S. C), tel que le calorimètre vendu sous la dénomination MDSC 2920 par la société TA instrument, avec une montée en température de 5 ou 10°C par minute, selon la norme ISO 11357-3:1999. L'enthalpie de fusion du composé pâteux est la quantité d'énergie nécessaire pour faire passer le composé de l'état solide à l'état liquide. Elle est exprimée eu J/g.

L'enthalpie de fusion consommée à 23°C est la quantité d'énergie absorbée par l'échantillon pour passer de l'état solide à l'état qu'il présente à 23°C constitué d'une fraction liquide et d'une fraction solide.

La fraction liquide du composé pâteux mesurée à 32°C représente de préférence de 30 à 100% en poids du composé, de préférence de 80 à 100%, de préférence encore de 90 à 100% en poids du composé. Lorsque la fraction liquide du composé pâteux mesurée à 32°C est égale à 100%, la température de la fin de la plage de fusion du composé pâteux est inférieure ou égale à 32°C.

La fraction liquide du composé pâteux mesurée à 32°C est égale au rapport de l'enthalpie de fusion consommée à 32°C sur l'enthalpie de fusion du composé pâteux. L'enthalpie de fusion consommée à 32°C est calculée de la même façon que l'enthalpie de fusion consommée à 23°C.

Les composés pâteux sont généralement des composés hydrocarbonés comme les lanolines et leurs dérivés ou encore des PDMS.

En particulier, le composé pâteux est de préférence choisi parmi les composés synthétiques et les composés d'origine végétale. Un composé pâteux peut être obtenu par synthèse à partir de produits de départ d'origine végétale.

La présence d'un composé pâteux peut permettre de conférer avantageusement un confort amélioré lors du dépôt d'une composition de l'invention sur les fibres kératiniques.

Un tel composé peut être avantageusement choisi parmi :
- la lanoline et ses dérivés,
- les composés siliconés polymériques ou non,
- les composés fluorés polymériques ou non,
- les polymères vinyliques, notamment :
   - les homopolymères d'oléfines,
   - les copolymères d'oléfines,
   - les homopolymères et copolymères de diènes hydrogénés,
   - les oligomères linéaires ou ramifiés, homo ou copolymères de (méth)acrylates d'alkyles ayant de préférence un groupement alkyle en C₈-C₃₀,
   - les oligomères homo et copolymères d'esters vinyliques ayant des groupements alkyle en C₈-C₃₀,
   - les oligomères homo et copolymères de vinyléthers ayant des groupements alkyle en C₈-C₃₀,
- les polyéthers liposolubles résultant de la polyéthérification entre un ou plusieurs diols en C₂-C₁₀₀, en particulier, en C₂-C₅₀,
- les esters d'acide ou d'alcool gras,
- et leurs mélanges.

Parmi les esters, on peut notamment citer :
- les esters d'un glycérol oligomère, notamment, les esters de diglycérol, en particulier les condensats d'acide adipique et de glycérol, pour lesquels une partie des groupes hydroxyle des glycérols ont réagi avec un mélange d'acides gras, tels que l'acide stéarique, l'acide caprique, l'acide stéarique et isostéarique et l'acide 12-hydroxystéarique, à l'image, notamment, de ceux commercialisé sous la marque Softisan 649 par la société Sasol ou tel que le polyacyladipate-2 de bis diglycéryle,
- le propionate d'arachidyle commercialisé sous la marque Waxenol 801 par Alzo,
- les esters de phytostérol,
- les triglycérides d'acides gras et leurs dérivés, tels que les coco-glycérides hydrogénés,
- les polyesters non réticulés résultant de la polycondensation entre un acide dicarboxylique ou un polyacide carboxylique linéaire ou ramifié en C₄-C₅₀ et un diol ou un polyol en C₂-C₅₀,
- les esters aliphatiques d'ester résultant de l'estérification d'un ester d'acide hydroxycarboxylique aliphatique par un acide carboxylique aliphatique (Salacos HCIS (V)-L commercialisé par la société Nishing Oil),
- les polyesters résultant de l'estérification, par un acide polycarboxylique, d'un ester d'acide hydroxy carboxylique aliphatique, ledit ester comprenant au moins deux groupes hydroxyle, tels que les produits Risocast DA-H^{®}, et Risocast DA-L^{®},
- et leurs mélanges.

Le composé pâteux peut également être choisi parmi les composés d'origine végétale. Parmi ceux-ci, on peut notamment citer la cire d'orange comme, par exemple, celle qui est commercalisée sous la référence Orange Peel Wax par la société Koster Keunen, le beurre de karité, l'huile d'olive partiellement hydrogénée comme, par exemple, le composé commercialisé sous la référence Beurrolive par la société Soliance ou encore le beurre de cacao.

Les composés pâteux peuvent être utilisés en une quantité allant de 1 à 50 % en poids, en particulier de 3 à 45 %, et plus particulièrement de 5 à 40 % en poids par rapport au poids total de la composition.

### Polymères semi-cristallins

Par "polymère semi-cristallin", on entend au sens de l'invention, des polymères comportant une partie cristallisable, chaîne pendante ou séquence dans le squelette, et une partie amorphe dans le squelette, et présentant une température de changement de phase réversible du premier ordre, en particulier de fusion (transition solide-liquide). Lorsque la partie cristallisable est une séquence du squelette polymérique, cette séquence cristallisable est de nature chimique différente de celle des séquences amorphes ; le polymère semi-cristallin est dans ce cas un polymère séquencé par exemple du type dibloc, tribloc ou multibloc.

De façon avantageuse, le ou les polymères semi-cristallins de la composition de l'invention ont une masse moléculaire moyenne en nombre M̅n supérieure ou égale à 2000, allant par exemple de 2 000 à 800 000, de préférence de 3 000 à 500 000, par exemple de 4 000 à 150 000, et mieux de 4 000 à 99 000.

Dans la composition selon l'invention, les polymères semi-cristallins sont avantageusement solubles dans la phase huileuse à au moins 1 % en poids, à une température supérieure à leur température de fusion. En dehors des chaînes ou séquences cristallisables, les séquences des polymères sont amorphes. Par "chaîne ou séquence cristallisable", on entend au sens de l'invention une chaîne ou séquence qui, si elle était seule, passerait de l'état amorphe à l'état cristallin, de façon réversible, selon qu'on est au-dessus ou en dessous de la température de fusion. Une chaîne au sens de l'invention est un groupement d'atomes, pendant ou latéral par rapport au squelette du polymère. Une séquence est un groupement d'atomes appartenant au squelette, groupement constituant un des motifs répétitifs du polymère.

De préférence, le squelette polymérique des polymères semi-cristallins est soluble dans la phase huileuse.

De façon préférée, les polymères semi-cristallins utilisés dans la composition de l'invention présentent une température de fusion (ou point de fusion), pF, inférieure à 70°C (25°C ≤pF< 70°C), cette température étant au moins égale à la température de la matière kératinique devant recevoir la composition selon l'invention, notamment la peau. La température de fusion peut être mesurée notamment par toute méthode connue et en particulier avec un calorimètre à balayage différentiel (D.S.C).

De préférence, les séquences ou chaînes cristallisables des polymères semi-cristallins représentent au moins 30 % du poids total de chaque polymère et mieux au moins 40 %. Les polymères semi-cristallins à séquences cristallisables utilisés selon l'invention sont des polymères séquencés ou multiséquencés. Ils peuvent être obtenus par polymérisation de monomères à doubles liaisons réactives (ou éthyléniques) ou par polycondensation. Lorsque les polymères de l'invention sont des polymères à chaînes latérales cristallisables, ils sont avantageusement sous forme aléatoire ou statistique.

Les polymères semi-cristallins de l'invention sont d'origine synthétique. En outre, ils ne comportent pas de squelette polysaccharidique.

Les polymères semi-cristallins utilisables dans l'invention sont de préférence choisis parmi les polymères (homopolymères ou copolymères) portant au moins une chaîne latérale cristallisable, et les polymères (homopolymères ou copolymères) portant dans le squelette au moins une séquence cristallisable, comme ceux décrits dans le document US-A-5,156,911. La ou les chaînes latérales ou séquences cristallisables sont hydrophobes.

Selon un mode préféré de réalisation de l'invention, les polymères semi-cristallins sont choisis notamment parmi les homopolymères et copolymères résultant de la polymérisation d'au moins un monomère à chaîne(s) cristallisable(s), celle-ci étant choisie parmi les chaînes alkyles comportant au moins 11 atomes de carbone et au plus 40 atomes de carbone et mieux au plus 24 atomes de carbone. Il s'agit notamment de chaînes alkyle comportant au moins 12 atomes de carbone, et de préférence, il s'agit de chaînes alkyle comportant de 14 à 24 atomes de carbone (C₁₄-C₂₄). Il peut s'agir de chaînes alkyle hydrocarbonées (atomes de carbone et d'hydrogène) ou chaînes alkyle fluorées ou perfluorées (atomes de carbone, atomes de fluor et éventuellement atomes d'hydrogène). Lorsqu'il s'agit de chaînes alkyle fluorées ou perfluorées, elles comportent au moins 11 atomes de carbone dont au moins 6 atomes de carbone sont fluorés.

Par "alkyle", on entend un groupement saturé (ne comportant pas d'insaturation).

Selon un mode particulier de réalisation de l'invention, le polymère semi-cristallin est choisi parmi les homopolymères obtenus par polymérisation d'au moins un monomère à chaîne cristallisable, choisi parmi les (méth)acrylates d'alkyle en C₁₄-C₂₄, les (méth)acrylates de perfluoroalkyle en C ₁₁ -C₁₅, les N alkyl (méth)acrylamides en C₁₄ à C₂₄ avec ou sans atome de fluor, les esters vinyliques à chaînes alkyle ou perfluoroalkyle en C₁₄ à C₂₄, les éthers vinyliques à chaînes alkyle ou perfluoroalkyle en C₁₄ à C₂₄, les alpha-oléfines en C₁₄ à C₂₄, les para-alkyl styrènes avec un groupe alkyle en C₁₄ à C₂₄, et parmi les copolymères de ces monomères, obtenus par copolymérisation de ces monomères avec un monomère hydrophile, de préférence différent de l'acide méthacrylique, comme par exemple la N-vinylpyrrolidone, l'hydroxyéthylacrylate, l'hydroxyéthylméthacrylate, l'acide acrylique. De tels copolymères peuvent être par exemple les copolymères d'alkyl-C₁₄-C₂₄-acrylate, d'alkyl-C₁₄₋C₂₄-méthacrylate, d'alkyl-C₁₄₋C₂₄-acrylamide, d'alkyl-C₁₄-C₂₄-méthacrylamide, avec la N-vinylpyrrolidone, l'hydroxyéthylacrylate, l'hydroxyéthyl-méthacrylate, l'acide acrylique, ou leurs mélanges.

De préférence, le polymère semi-cristallin est choisi parmi les homopolymère obtenus par polymérisation d'un monomère choisi parmi les acrylates d'alkyle en C₁₄-C₂₄ et les méthacrylates d'alkyle en C₁₄-C₂₄ et parmi les copolymères obtenus par copolymérisation d'un monomère choisi parmi les acrylates d'alkyle en C₁₄-C₂₄ et les méthacrylates d'alkyle en C₁₄-C₂₄, avec un monomère hydrophile tel que l'acide acrylique.

Les polymères semi-cristallins de la composition de l'invention peuvent être non réticulés ou réticulés en partie, du moment que le taux de réticulation ne gène pas leur dissolution ou dispersion dans la phase huileuse par chauffage au-dessus de leur température de fusion. Il peut s'agir alors d'une réticulation chimique, par réaction avec un monomère multifonctionnel lors de la polymérisation. Il peut aussi s'agir d'une réticulation physique qui peut alors être due soit à l'établissement de liaisons type hydrogène ou dipolaire entre des groupes portés par le polymère, comme par exemple les interactions dipolaires entre ionomères carboxylates, ces interactions étant en faible quantité et portées par le squelette du polymère ; soit à une séparation de phase entre les séquences cristallisables et les séquences amorphes portées par le polymère.

De préférence, les polymères semi-cristallins de la composition selon l'invention sont non réticulés.

Selon un mode particulier de réalisation de l'invention, le polymère semi-cristallin est un homopolymère résultant de la polymérisation d'un monomère à chaîne cristallisable choisi parmi les acrylates d'alkyle en C₁₄-C₂₄ et les méthacrylates d'alkyle en C₁₄-C₂₄. On peut citer notamment ceux commercialisés sous les dénominations Intelimer® par la société Landec, décrits dans la brochure "Intelimer® polymers", Landec IP22. Ces polymères sont sous forme solide à température ambiante. Ils portent des chaînes latérales cristallisables et correspondent à des homopolymères d'acrylates ou méthacrylates d'alkyle en C₁₄-C₂₄ saturé. On peut citer plus particulièrement l'homopolymère d'acrylate de stéaryle (Intelimer IPA-13.1) (nom INCI : Poly C10-30 alkyl acrylate), l'homopolymère d'acrylate de béhényle (Intelimer IPA-13.6) (nom INCI : Poly C10-30 alkyl acrylate).

Selon un autre mode particulier de réalisation de l'invention, le polymère semi-cristallin est un copolymère d'acrylates d'alkyle en C₁₄-C₂₄ ou de méthacrylates d'alkyle en C₁₄-C₂₄ avec l'acide acrylique. Comme copolymères de ce type, on peut citer les copolymères obtenus par la copolymérisation de l'acrylate de béhényle et de l'acide acrylique, et les copolymères obtenus par la copolymérisation de l'acrylate de stéaryle et de l'acide acrylique.

Selon un mode préféré de réalisation de l'invention, le polymère semi-cristallin est un homopolymère, et il est choisi parmi l'homopolymère d'acrylate de stéaryle (Intelimer IPA-13.1) (nom INCI : Poly C10-30 alkyl acrylate), l'homopolymère d'acrylate de béhényle (Intelimer IPA-13.6) (nom INCI : Poly C10-30 alkyl acrylate), et leurs mélanges.

Les polymères semi-cristallins peuvent être utilisés en une quantité allant de 1 à 50 % en poids, en particulier de 3 à 45 %, et plus particulièrement de 5 à 40 % en poids par rapport au poids total de la composition.

### Gélifiants lipophiles

Les gélifiants utilisables dans les compositions selon l'invention peuvent être des gélifiants lipophiles organiques ou minéraux, polymériques ou moléculaires.

Comme gélifiant lipophile minéral, on peut citer les argiles éventuellement modifiées comme les hectorites modifiées par un chlorure d'ammonium d'acide gras en C₁₀ à C₂₂, comme l'hectorite modifiée par du chlorure de di-stéaryl di-méthyl ammonium telle que, par exemple, celle commercialisée sous la dénomination de Bentone 38V^{Ⓡ} par la société ELEMENTIS.

On peut également citer la silice pyrogénée éventuellement traitée hydrophobe en surface dont la taille des particules est inférieure à 1 µm Il est en effet possible de modifier chimiquement la surface de la silice, par réaction chimique générant une diminution du nombre de groupes silanol présents à la surface de la silice. On peut notamment substituer des groupes silanol par des groupements hydrophobes : on obtient alors une silice hydrophobe. Les groupements hydrophobes peuvent être :
- des groupements triméthylsiloxyle, qui sont notamment obtenus par traitement de silice pyrogénée en présence de l'hexaméthyldisilazane. Des silices ainsi traitées sont dénommées « Silica silylate » selon le CTFA (6ème édition, 1995). Elles sont par exemple commercialisées sous les références Aerosil R812^{Ⓡ} par la société DEGUSSA, CAB-O-SIL TS-530^{Ⓡ} par la société CABOT,
- des groupements diméthylsilyloxyle ou polydiméthylsiloxane, qui sont notamment obtenus par traitement de silice pyrogénée en présence de polydiméthylsiloxane ou du diméthyldichlorosilane. Des silices ainsi traitées sont dénommées "Silica diméthyl silylate" selon le CTFA (6ème édition, 1995). Elles sont par exemple commercialisées sous les références Aerosil R972^{Ⓡ}, et Aerosil R974^{Ⓡ} par la société DEGUSSA, CAB-O-SIL TS-610^{Ⓡ} et CAB-O-SIL TS-720^{Ⓡ} par la société CABOT.

La silice pyrogénée hydrophobe présente en particulier une taille de particules pouvant être nanométrique à micrométrique, par exemple allant d'environ de 5 à 200 nm.

Les gélifiants lipophiles organiques polymériques sont par exemple les organopolysiloxanes élastomériques partiellement ou totalement réticulés, de structure tridimensionnelle, comme ceux commercialisés sous les dénominations de KSG6^{Ⓡ}, KSG16^{Ⓡ} et de KSG18^{Ⓡ} par la société SHIN-ETSU, de Trefil E-505C^{Ⓡ} et Trefil E-506C^{Ⓡ} par la société DOW-CORNING, de Gransil SR-CYC^{Ⓡ}, SR DMF10^{Ⓡ}, SR-DC556^{Ⓡ}, SR 5CYC gel^{Ⓡ}, SR DMF 10 gel^{Ⓡ} et de SR DC 556 gel^{Ⓡ} par la société GRANT INDUSTRIES, de SF 1204^{Ⓡ} et de JK 113^{Ⓡ} par la société GENERAL ELECTRIC ; l'éthylcellulose comme celle vendue sous la dénomination Ethocel^{Ⓡ} par la société DOW CHEMICAL ; les polycondensats de type polyamide résultant de la condensation entre (α) au moins un acide choisi parmi les acides dicarboxyliques comprenant au moins 32 atomes de carbone tels que les acides gras dimères et (β) un alkylène diamine et en particulier l'éthylène diamine, dans lequel le polymère polyamide comprend au moins un groupe acide carboxylique terminal estérifié ou amidifié avec au moins un mono alcool ou une mono amine comprenant de 12 à 30 atomes de carbone linéaires et saturés, et en particulier, les copolymères d'éthylène diamine/dilinoléate de stéaryle tel que celui commercialisé sous la dénomination Uniclear 100 VG^{®} par la société ARIZONA CHEMICAL ; les polyamides siliconés du type polyorganosiloxane tels que ceux décrits dans les documents US-A-5 874 069, US-A-5,919,441, US-A-6,051,216 et US-A-5,981,680 comme par exemple ceux commercialisés sous la référence Dow Corning 2-8179 et Dow Corning 2-8178 Gellant Gellant par la société DOW CORNING ; les galactommananes comportant de un à six, et en particulier de deux à quatre, groupes hydroxyle par ose, substitués par une chaîne alkyle saturée ou non, comme la gomme de guar alkylée par des chaînes alkyle en C₁ à C₆, et en particulier en C₁ à C₃ et leurs mélanges. Les copolymères séquencés de type "dibloc", "tribloc" ou "radial" du type polystyrène/polyisoprène, polystyrène/polybutadiène tels que ceux commercialisés sous la dénomination Luvitol HSB^{®} par la société BASF, du type polystyrène/copoly(éthylène-propylène) tels que ceux commercialisés sous la dénomination de Kratori^{Ⓡ} par la société SHELL CHEMICAL CO ou encore du type polystyrène/copoly(éthylène-butylène), les mélanges de copolymères tribloc et radial (en étoile) dans l'isododécane tels que ceux commercialisé par la société PENRECO sous la dénomination Versagel^{®} comme par exemple le mélange de copolymère tribloc butylène/éthylène/styrène et de copolymère étoile éthylène/propylène/styrène dans l'isododécane (Versagel M 5960).

Les compositions selon l'invention peuvent comprendre également en tant que gélifiant lipophile un élastomère de silicone non émulsionnant. Des élastomères non-émulsionnants sont notamment décrits dans les demandes JP-A-61-194009, EP-A-242219, EP-A-285886, EP-A-765656. Comme élastomères non-émulsionnants sphériques, on peut utiliser ceux vendus sous les dénominations "DC 9040", "DC9041", "DC 9509", "DC9505", "DC 9506" par la société Dow Corning. L'élastomère de silicone non émulsionnant sphérique peut se présenter également sous forme de poudre d'organopolysiloxane réticulé élastomère enrobée de résine de silicone, notamment de résine silsesquioxane, comme décrit par exemple dans le brevet US5538793. De tels élastomères sont vendus sous les dénominations "KSP-100", "KSP-101", "KSP-102", "KSP-103", KSP-104", "KSP-105" par la société Shin Etsu.

D'autres organopolysiloxanes réticulés élastomères sous forme de poudres sphériques peuvent être des poudres de silicone hybride fonctionnalisé par des groupes fluoroalkyle, notamment vendues sous la dénomination "KSP-200" par la société Shin Etsu ; des poudres de silicones hybride fonctionnalisé par des groupes phényl, notamment vendues sous la dénomination "KSP-300" par la société Shin Etsu.

On peut également utiliser dans les compositions selon l'invention des élastomères de silicones avec groupement MQ, tels que ceux vendus par la Société Wacker sous les dénominations Belsil RG100, Belsil RPG33 et préférenciellement RG80. Ces élastomères particuliers, lorsqu'ils sont en association avec les résines selon l'invention, peuvent permettre d'améliorer les propriétés de non transfert des compositions les comprenant.

On peut encore citer parmi les gélifiants lipophiles les organogélateurs et notamment :
- Les dérivés de bis-urées de formule générale (I) : 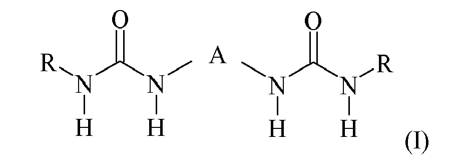 dans laquelle :
- A est un groupement de formule : 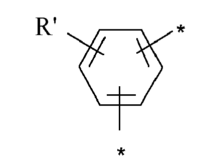 avec R' étant un radical alkyle C₁ à C₄ linéaire ou ramifié et les * symbolisant les points de rattachement du groupement A à chacun des deux atomes d'azote du reste du composé de formule générale (I), et
- R est un radical alkyle en C₆ à C₁₅, mono-ramifié, non cyclique, saturé ou insaturé et dont la chaîne hydrocarbonée est éventuellement interrompue par 1 à 3 hétéroatomes choisis parmi O, S et N, ou
   l'un de ses sels ou isomères notamment décrits dans la demande de brevet FR-A-2892303
- Les dérivés de bis-urées siliconés de formule générale (I) ou l'un de ses sels et/ou isomères : 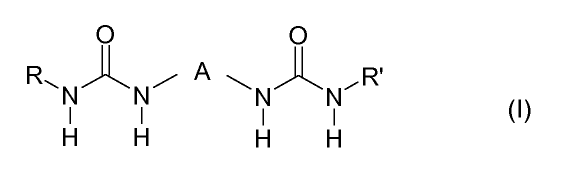 dans laquelle :
- A est un groupement de formule (II) : 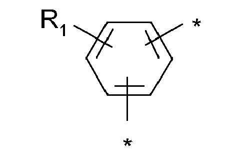 avec R1 étant un radical alkyle C₁ à C₄ linéaire ou ramifié, et les * symbolisant les points de rattachement du groupement A à chacun des deux atomes d'azote du reste du composé de formule générale (I), et
- R et R', identiques ou différents, sont choisis parmi :
- i) les radicaux de formule (III) : 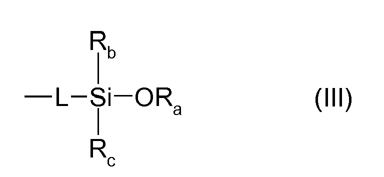 dans laquelle :
- L est une liaison simple ou un radical divalent carboné, notamment hydrocarboné (alkylène), linéaire, ramifié et/ou cyclique, saturé ou insaturé, comprenant 1 à 18 atomes de carbone, et pouvant comprendre 1 à 4 hétéroatomes choisis parmi N, O et S;
- Ra est :
   a) un radical carboné, notamment hydrocarboné (alkyle), linéaire, ramifié et/ou cyclique, saturé ou insaturé, comprenant 1 à 18 atomes de carbone, et pouvant comprendre 1 à 8 hétéroatomes choisis parmi N, O, Si et S; ou bien
   b) un radical siliconé de formule : 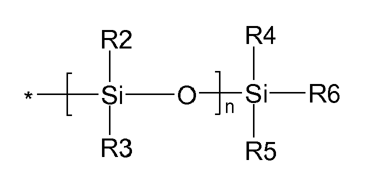 avec n étant compris entre 0 et 100, notamment entre 1 et 80, voire 2 à 20;
      et R2 à R6 étant, indépendamment les uns des autres, des radicaux carbonés, notamment hydrocarbonés (alkyles) linéaires ou ramifiés, ayant 1 à 12, notamment 1 à 6 atomes de carbone, et pouvant comprendre 1 à 4 hétéroatomes, notamment O;
- Rb et Rc sont, indépendamment l'un de l'autre, choisis parmi:
   a) les radicaux carbonés, notamment hydrocarbonés (alkyles), linéaires, ramifiés et/ou cycliques, saturés ou insaturés, comprenant 1 à 18 atomes de carbone, et pouvant comprendre 1 à 4 hétéroatomes choisis parmi N, O, Si et S;
   b) les radicaux de formule : 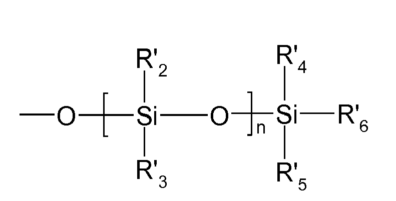 avec n étant compris entre 0 et 100, notamment entre 1 et 80, voire 2 à 20;
      et R'2 à R'6 étant, indépendamment les uns des autres, des radicaux carbonés, notamment hydrocarbonés (alkyles) linéaires ou ramifiés, ayant 1 à 12, notamment 1 à 6 atomes de carbone, et pouvant comprendre 1 à 4 hétéroatomes, notamment O.
      et
- ii) les radicaux alkyle en C₁ à C₃₀, linéaire, ramifié et/ou cyclique, saturé ou insaturé, et comprenant éventuellement 1 à 3 hétéroatomes choisis parmi O, S, F et N;
   étant entendu que l'un au moins des radicaux R et/ou R' est de formule (III) tels que ceux décrits dans la demande de brevet FR-A-2900819.
   - Les dérivés de bis-urées décrits dans la demande de brevet FR-A-2894476.

Parmi les autres gélifiants lipophiles pouvant être utilisés dans les compositions selon l'invention, on peut encore citer les alcools gras comprenant de 10 à 30 atomes de carbone. Par alcool gras comprenant de 10 à 30 atomes de carbone, on entend tout alcool gras pur, saturé ou non, ramifié ou non, comportant de 10 à 30 atomes de carbone.

On utilise de préférence un alcool gras comprenant de 10 à 26 atomes de carbone, mieux de 10 à 24 atomes de carbone et encore mieux de 14 à 22 atomes de carbone.

On peut citer notamment comme alcools gras utilisables, les alcools laurique, myristique, cétylique, stéarylique, oléique, cétéarylique (mélange d'alcool cétylique et stéarylique), béhénique, érucique et leurs mélanges. On utilise de préférence l'alcool cétylique ou béhénique.

De tels alcools gras sont notamment commercialisés sous la dénomination NAFOL par la société SASOL.

Parmi les gélifiants lipophiles pouvant être utilisés dans les compositions selon l'invention, on peut encore citer les esters de dextrine et d'acide gras, tels que les palmitates de dextrine, notamment tels que ceux commercialisés sous les dénominations Rheopearl TL^{Ⓡ} ou Rheopearl KL^{Ⓡ} par la société CHIBA FLOUR.

Les gélifiants lipophiles peuvent être utilisés en une quantité allant de 1 à 60 % en poids, en particulier de 2 à 50 %, et plus particulièrement de 5 à 40 % en poids par rapport au poids total de la composition.

### ADDITIFS

### Matière colorante

Selon l'invention ou selon un mode de réalisation avantageux de l'invention, selon l'objet de l'invention considéré, les compositions de maquillage comprennent au moins une matière colorante choisie parmi les matières pulvérulentes.

Les matières colorantes pulvérulentes peuvent être choisies parmi les pigments et les nacres.

Les pigments peuvent être blancs ou colorés, minéraux et/ou organiques, enrobés ou non. On peut citer, parmi les pigments minéraux, le dioxyde de titane, éventuellement traité en surface, les oxydes de zirconium, de zinc ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les nacres comprennent des particules colorées de toute forme, irisées ou non, notamment produites par certains mollusques dans leur coquille ou bien synthétisées et qui présentent un effet de couleur par interférence optique. Comme exemples de nacres, on peut citer les pigments nacrés tels que le mica titane recouvert avec un oxyde de fer, le mica recouvert d'oxychlorure de bismuth, le mica titane recouvert avec de l'oxyde de chrome, les pigments nacrés à base d'oxychlorure de bismuth. Il peut également s'agir de particules de mica à la surface desquelles sont superposées au moins deux couches successives d'oxydes métalliques et/ou de matières colorantes organiques. Les nacres peuvent plus particulièrement posséder une couleur ou un reflet jaune, rose, rouge, bronze, orangé, brun, or et/ou cuivré..

Les compositions selon l'invention peuvent également comprendre en outre au moins une matière colorante choisie parmi les colorants liposolubles, les colorants hydrosolubles.

Les colorants liposolubles sont par exemple le rouge Soudan, le D&C Red 17, le D&C Green 6, le β-carotène, l'huile de soja, le brun Soudan, le D&C Yellow 11, le D&C Violet 2, le D&C Orange 5, le jaune quinoléine, le rocou.

Les matières pulvérulentes peuvent être présentes en une teneur allant de 1 à 15% en poids par rapport au poids total de la composition, de préférence de 5 à 10% en poids.

De manière générale, ces matières colorantes peuvent être présentes en une teneur allant de 0,01 à 30 % en poids par rapport au poids total de la composition.

Selon un mode particulier, les oxydes de fer seront présents en une teneur allant de 0,01 à 15% en poids, de préférence de 0,01 à 10% en poids par rapport au poids total de ladite composition.

### Fibres

Les compositions conformes à l'invention peuvent en outre comprendre des fibres qui permettent une amélioration de l'effet allongeant.

Par "fibre", il faut comprendre un objet de longueur L et de diamètre D tel que L soit très supérieur à D, D étant le diamètre du cercle dans lequel s'inscrit la section de la fibre. En particulier, le rapport L/D (ou facteur de forme) est choisi dans la gamme allant de 3,5 à 2500, en particulier de 5 à 500, et plus particulièrement de 5 à 150.

Les fibres utilisables dans la composition de l'invention peuvent être des fibres d'origine synthétique ou naturelle, minérale ou organique. Elles peuvent être courtes ou longues, unitaires ou organisées par exemple tressées, creuses ou pleines. Leur forme peut être quelconque et notamment de section circulaire ou polygonale (carrée, hexagonale ou octogonale) selon l'application spécifique envisagée. En particulier, leurs extrémités sont épointées et/ou polies pour éviter de se blesser.

En particulier, les fibres ont une longueur allant de 1 µm à 10 mm, en particulier de 0,1 mm à 5 mm et plus particulièrement de 0,3 mm à 3,5 mm. Leur section peut être comprise dans un cercle de diamètre allant de 2 nm à 500 µm, en particulier allant de 100 nm à 100 µm et plus particulièrement de 1 µm à 50 µm . Le poids ou titre des fibres est souvent donné en denier ou décitex et représente le poids en gramme pour 9 km de fil. Les fibres selon l'invention peuvent en particulier avoir un titre choisi dans la gamme allant de 0,15 à 30 deniers et notamment de 0,18 à 18 deniers.

Les fibres utilisables dans la composition de l'invention peuvent être choisies parmi les fibres rigides ou non rigides, elles peuvent être d'origine synthétique ou naturelle, minérales ou organiques.

Par ailleurs, les fibres peuvent être traitées ou non en surface, enrobées ou non, colorées ou non colorées.

A titre de fibres utilisables dans la composition selon l'invention, on peut citer les fibres non rigides telles que les fibres de polyamide (Nylon®) ou les fibres rigides telles que les fibres de polyimide-amide comme celles vendues sous les dénominations KERMEL^{Ⓡ}, KERMEL TECH^{Ⓡ} par la société RHODIA ou de poly-(p-phénylène-téréphtalamide) (ou d'aramide) notamment vendues sous la dénomination Kevlar ® par la société DUPONT DE NEMOURS.

Les fibres peuvent êtres présentes dans la composition selon l'invention en une teneur allant de 0,01 % à 10 % en poids, par rapport au poids total de la composition, en particulier de 0,1 % à 5 % en poids, et plus particulièrement de 0,3 % à 3 % en poids.

### Actifs cosmétiques

Comme actifs cosmétiques pouvant être utilisés dans les compositions conformes à l'invention, on peut citer notamment des antioxydants, les conservateurs, les parfums, les neutralisants, émollients, des hydratants, des vitamines et des filtres en particulier solaires.

Bien entendu, l'homme du métier veillera à choisir les éventuels additifs complémentaires et/ou leur quantité de telle manière que les propriétés avantageuses de la composition selon l'invention ne soient pas, ou substantiellement pas, altérées par l'adjonction envisagée.

### COMPOSITION DE DEMAQUILLAGE ET/OU DE NETTOYAGE

La composition de démaquillage et/ou de nettoyage peut comprendre majoritairement de l'eau et/ou un solvant hydrosoluble, par exemple en une teneur supérieure ou égale à 50%, mieux supérieure ou égale à 80% en poids par rapport au poids total de ladite composition.

Afin de réaliser ce démaquillage, au moins un doigt peut être imprégné de cette composition. En variante, un support peut être à imprégner et ainsi être sec ou anhydre. En variante, ce support peut être pré-imprégné de la composition.

Ce support peut être un tissé ou un non tissé, éventuellement laminé, tel qu'un disque demakeup®.

### KIT DE MAQUILLAGE ET DE DEMAQUILLAGE ET/OU NETTOYAGE

Ce kit peut comprendre une composition de maquillage selon l'invention en association avec une composition de démaquillage et/ou de nettoyage.

Cette composition de démaquillage et/ou de nettoyage peu(ven)t être conditionnés avec ladite composition de maquillage, séparément, dans un même article de conditionnement.

La composition de maquillage peut être un produit de maquillage pour les lèvres tel qu'un rouge à lèvres, un baume à lèvres, un brillant à lèvres ou un crayon à lèvres, un produit de teint, tel qu'un fond de teint, une poudre libre ou compactée, un fard à joues ou à paupières, un produit anti-cernes, un blush, un mascara, un eye-liner ou encore un produit de maquillage du corps ou de coloration de la peau.

La composition de démaquillage et/ou nettoyage peut être telle que précédemment décrite.

### EXEMPLES relatifs à l'utilisation d'un système émulsionnant particulier

Des formulations de mascara sont préparées en utilisant de l'eau désionisée comme suit.

### Exemple 1 : Composition hors invention

| Ingrédients | % |
|---|---|
| eau | 45,5 |
| Hydroxyéthylcellulose (HEC) | 0,7 |
| Triéthanolamine (TEA) | 2 |
| glyceryl stéarate | 2,5 |
| | |
| Cire d'abeille | 10 |
| acide stearique | 5 |
| conservateurs | 0,3 |
| | |
| oxyde de fer noir | 10 |
| Simethicone | 0,5 |
| Dispersion de polyurethane dans l'eau à 43% de matière active | 23,5 |

On fait fondre à 95°C la cire d'abeille avec les conservateurs et l'acide stearique. Puis l'oxyde de fer noir est dispersé sous Moritz. Dans de l'eau portée à 95°C est ensuite dispersé sous Rayneri l'hydroxéthylcellulose (HEC) puis la triéthanolamine et le glyceryl stéarate. Sous agitateur Moritz, la phase aqueuse est ajoutée sur la phase grasse jusqu'à formation de l'émulsion. Cette émulsion est refroidie sous agitation lente. Vers 30°C est ajouté doucement le polyuréthane.

Produits utilisés dans cet exemple :
- Le Glyceryl stéarate vendu sous l'appellation TEGIN M PELLETS par Evonic Goldschmidt Gmbh, Essen,
- Le Simethicone vendu sous l'appellation MIRASIL SM par Rhodia Silicones S.A.S., Lyon,
- le Polyuréthane dispersé dans l'eau est vendu sous l'appellation BAYCUSAN C 1000 par Bayer MaterialScience LLC, Pittsburgh, PA,

Les autres ingrédients étant aisément accessibles pour un homme du métier.

Le résultat du test de démaquillage est l'absence de gaines.

### Exemple 2 : Composition 1 selon l'invention

| Ingrédients | % |
|---|---|
| eau | 36,48 |
| Hydroxyéthylcellulose (HEC) | 0,7 |
| | |
| Cire d'abeille | 10 |
| Alcool cétylique | 2 |
| Alcool stearylique 20(OE) | 4,44 |
| Alcool stearylique 2(OE) | 2,1 |
| Potassium cetyl phosphate | 2,18 |
| Conservateur | 0,3 |
| | |
| Oxyde de fer noir | 10 |
| Simethicone | 0,5 |
| Dispersion de polyuréthane dans l'eau à 32% de matière active | 31,3 |

Cette composition est préparée de la même façon que précédemment décrite, seul le système émulsionnant changeant, le système tensioactif étant remplacé par un système émulsionnant comprenant de l'Alcool cétylique, de l'Alcool stearylique 20 OE, de l'Alcool stearylique 2 OE et du Potassium cetyl phosphate.

Produits utilisés dans cet exemple :
- L'Alcool cétylique vendu sous l'appellation PHYTOWAX ricin 16L64 et 22L73 par la société SOPHIM,
- L'Alcool stearylique 20(OE) vendu sous l'appellation BRIJ 78 par la société

### UNIQEMA,

- L'Alcool stearylique 2(OE) vendu sous l'appellation BRIJ 72 commercialisé par la société UNIQEMA,
- Le Potassium cetyl phosphate vendu sous l'appellation Amphisol K par

### GIVAUDAN,

- Le Simethicone vendu sous l'appellation MIRASIL SM par Rhodia Silicones S.A.S., Lyon,
- le Polyuréthane dispersé dans l'eau est vendu sous l'appellation BAYCUSAN C 1001 par Bayer MaterialScience LLC, Pittsburgh, PA,

Les autres ingrédients étant aisément accessibles pour un homme du métier.

Le résultat du test de démaquillage avec une telle composition selon le protocole décrit avant est un démaquillage par gaines.

### Exemple 3 : Composition 2 selon l'invention

| **Ingrédients** | % |
|---|---|
| eau | 41,7 |
| Parabène | 0,25 |
| Phénoxyethanol et parabens | 1,2 |
| Hydroxybenzoate | 0,15 |
| EDTA | 0,2 |
| Dehydroacetate de sodium | 0,2 |
| PEG 200 glyceryl stéarate | 4 |
| 1,3 Butylene glycol | 5 |
| Pigment noir | 7 |
| Copolymere acrylamide | 2,5 |
| Cire d'abeille | 7,4 |
| Cire de carnauba | 3,5 |
| Parabène | 0,05 |
| Simethicone | 0,1 |
| Dispersion de polyuréthane dans l'eau à 32% de matière active | 23,75 |
| éthanol | 3 |

Cette formulation est préparée comme suit :
- Chauffer l'eau à 95°C et disperser sous Rayneri les conservateurs, le tensio-actif, le Butylène Glycol puis les pigments. Ajouter le copolymère acrylamide.
- Fondre la phase grasse à 95°C. Ajouter sur la phase aqueuse sous agitateur Rayneri, 10 minutes d'agitation puis laisser refroidir sous agitation lente. Vers 30°C, ajouter doucement le polyuréthane puis méthanol.

Produits utilisés dans cet exemple :
- le PEG 200 glyceryl stéarate vendu sous l'appellation SIMULSOL 220 par la société SEPIC S.A., Paris,
- le 1,3-Butylène glycol vendu par exemple par la société CELANESE CHEMICAL, Dallas,
- le Copolymère acrylamide vendu sous l'appellation SIMULGEL 600 par la société SEPPIC S.A., Paris,
- le Siméthicone vendu sous l'appellation MIRASIL SM par la société

### RHODIA SILICONES,

- le Polyuréthane dispersé dans l'eau est vendu sous l'appellation BAYCUSAN C 1001 par Bayer MaterialScience LLC, Pittsburgh, PA,

Les autres ingrédients étant aisément accessibles pour un homme du métier.

Le résultat du test de démaquillage avec une telle composition est un démaquillage par gaines.

### EXEMPLES relatifs à la mise en oeuvre d'un épaississant hydrophile particulier

### Protocoles :

Afin d'évaluer la « tenue à l'eau » in vitro d'une composition, on évalue cette dernière selon le protocole suivant :
- On applique la composition sur 3 éprouvettes de cheveux caucasiens raides 30 noeuds (60 cils longs de 1 cm) longueur de la frange 2 cm en faisant 3 x 10 passages espacés de 2 minutes avec reprise de produit entre chaque série de 10. Chaque éprouvette est ensuite séchée à température ambiante pendant un temps de séchage d'une heure.

Les 3 éprouvettes maquillées sont immergées dans un récipient contenant de l'eau à 20°C pendant un temps donné (1 heure, 24 heures ou 1 semaine). On effectue ensuite 5 allers-retours des 3 éprouvettes sur une chiffonnette carrée type Wypall L40 de chez Kimberly-clark.
- On évalue alors la présence de trace déposée par l'éprouvette.
- On attribue aux traces résultantes un score compris entre 0 et 9 ; 0 étant le score obtenu lorsqu'aucune trace n'est déposée par l'éprouvette, et 9 le score obtenu lorsque des traces très importantes sont déposées.

Par « tenue au sébum », on entend selon la présente demande la tenue au sébum in vitro évaluée selon le même protocole de mesure que pour la tenue à l'eau décrit ci-dessus, à la différence que les 3 éprouvettes maquillées sont immergées dans un récipient contenant du squalène (le squalène est présent à 18% dans la composition du sébum), au lieu d'eau.

Par « résistance aux frottements », on entend selon la présente demande la résistance aux frottements in vitro évaluée selon le protocole suivant :
- On applique la composition sur 3 éprouvettes de cheveux caucasiens raides 30 noeuds (60 cils longs de 1 cm) longueur de la frange 2 cm en faisant 3 x 10 passages espacés de 2 minutes avec reprise de produit entre chaque série de 10. Chaque éprouvette est ensuite séchée à température ambiante pendant un temps de séchage d'une heure.
- L'éprouvette maquillée est ensuite positionnée perpendiculairement au-dessus d'une feuille de papier et frottée à l'aide d'une brosse dure type Keracils ® (30 passages). On évalue la quantité de grains ainsi formés, récupérés sur la feuille de papier.
- On attribue à cette quantité de grains un score compris entre 0 et 6 ; 0 étant le score pour lequel aucun grain n'est récupéré sur la feuille de papier, et 6 le score pour lequel une quantité très importante de grains est récupérée.

Des formulations de mascara utilisant de l'eau désionisée sont préparées comme suit.

### Exemple 1 : Composition comparative

| **Ingrédients** | **%** |
|---|---|
| eau | 42,7 |
| Parabène | 0,25 |
| Mélange phénoxyethanol et parabens | 1,2 |
| Hydroxybenzoate | 0,15 |
| EDTA | 0,2 |
| Dehydroacetate de sodium | 0,2 |
| Hydroxyethylcellulose (HEC) | 1 |
| PEG 200 Stearate de glyceryl | 4 |
| 1,3-Butylene glycol | 5 |
| Pigment noir | 7 |
| polysorbate80 | 0,5 |
| Cire d'abeille | 7,4 |
| Carnauba | 3,5 |
| Parabène | 0,05 |
| Simethicone | 0,1 |
| latex | 23,75 |
| éthanol | 3 |

Cette formulation est préparée comme suit :
- Chauffer l'eau à 95°C et disperser sous Rayneri les conservateurs, le tensio-actif et l'HEC, le Butylène Glycol puis les pigments. Ajouter le copolymère acrylamide.
- Fondre la phase grasse à 95°C. Ajouter sur la phase aqueuse sous agitateur Rayneri, 10 minutes d'agitation puis laisser refroidir sous agitation lente. Vers 30°C, ajouter doucement le polyuréthane puis l'éthanol.

Produits utilisés dans cet exemple :
- le PEG 200 glyceryl stéarate vendu sous l'appellation SIMULSOL 220 par la société SEPPIC S.A., Paris,
- le 1,3-Butylène glycol vendu par exemple par la société CELANESE CHEMICAL, Dallas,
- le Siméthicone vendu sous l'appellation MIRASIL SM par la société RHODIA SILICONES,
- le Polyuréthane dispersé dans l'eau est vendu sous l'appellation BAYCUSAN C 1001 par Bayer MaterialScience LLC, Pittsburgh, PA,

Les autres ingrédients étant aisément accessibles pour un homme du métier.

Les protocoles d'évaluation de tenue précédemment décrits ont donnés avec cette composition les résultats suivants :

| | | | |
|---|---|---|---|
| tenue à l'eau 1min | à l'eau 1h | au sébum 1h | à sec |
| 2 | 3 | 1 | 5 |

### Exemple 2 : Composition avec épaississant hydrophile

| **Ingrédients** | **%** |
|---|---|
| eau | 43,4 |
| Parabène | 0,25 |
| Mélange phénoxyethanol et parabens | 1,2 |
| Hydroxybenzoate | 0,15 |
| EDTA | 0,2 |
| Dehydroacetate de sodium | 0,2 |
| PEG 200 Stéarate de glycéryle | 4 |
| 1,3-Butylene glycol | 5 |
| Pigment noir | 7 |
| Epaississant hydrophile | 0,8 |
| Cire d'abeille | 7,4 |
| Carnauba | 3,5 |
| Parabène | 0,05 |
| Simethicone | 0,1 |
| latex | 23,75 |
| éthanol | 3 |

- Chauffer l'eau à 95°C et disperser sous Rayneri les conservateurs, le tensio-actif, le Butylène Glycol puis les pigments. Ajouter le copolymère acrylamide.
- Fondre la phase grasse à 95°C. Ajouter sur la phase aqueuse sous agitateur Rayneri, 10 minutes d'agitation puis laisser refroidir sous agitation lente. Vers 30°C, ajouter doucement le polyuréthane puis méthanol.

Produits utilisés dans cet exemple :
- le PEG 200 Stéarate de glycéryle vendu sous l'appellation SIMULSOL 220 par la société SEPPIC S.A., Paris,
- le 1,3-Butylène glycol vendu par exemple par la société CELANESE CHEMICAL, Dallas,
- l'épaississant hydrophile vendu sous l'appellation PEMULEN TR2 par la société Lubrizol,
- le Siméthicone vendu sous l'appellation MIRASIL SM par la société RHODIA SILICONES,
- le Polyuréthane dispersé dans l'eau est vendu sous l'appellation BAYCUSAN C 1001 par Bayer MaterialScience LLC, Pittsburgh, PA,

Les autres ingrédients étant aisément accessibles pour un homme du métier.

### Résultats

Les protocoles d'évaluation de tenue précédemment décrits ont donnés avec cette composition les résultats suivants :

| | | | |
|---|---|---|---|
| tenue à l'eau 1min | à l'eau 1h | au sébum 1h | à sec |
| 0 | 0 | 1 | 4 |

On observe ainsi en comparant les deux compositions que la composition selon l'invention renfermant un épaississant hydrophile du type Pemulen améliore considérablement la tenue à l'eau, qui est excellente et pérenne.

La composition selon l'invention conserve en outre une excellente tenue au sébum.

Cette composition présente par ailleurs une bonne résistance aux frottements, qui est améliorée.

### EXEMPLES relatifs à la mise en oeuvre d'un plastifiant lipophile

### Protocole

Les mêmes protocoles précédemment décrits sont appliqués aux exemples qui suit en vue d'évaluer la tenue à l'eau, la tenue au sébum et la résistance aux frottements des compositions testées.

Des formulations de mascara sont préparées en utilisant de l'eau désionisée comme suit.

### Exemple 1 : Composition comparative

| **Ingrédients** | **%** |
|---|---|
| eau | 42,7 |
| Parabène | 0,25 |
| Mélange phénoxyethanol et parabens | 1,2 |
| Hydroxybenzoate | 0,15 |
| EDTA | 0,2 |
| Dehydroacetate de sodium | 0,2 |
| HEC | 1 |
| PEG 200 Stéarate de glycéryle | 4 |
| 1,3-Butylene glycol | 5 |
| Pigment noir | 7 |
| Polysorbate 80 | 0,5 |
| Cire d'abeille | 7,4 |
| Carnauba | 3,5 |
| Parabène | 0,05 |
| Simethicone | 0,1 |
| Dispersion de polyuréthane dans l'eau à 32% de matière active | 23,75 |
| éthanol | 3 |

Cette formulation est préparée comme suit :
- Chauffer l'eau à 95°C et disperser sous Rayneri les conservateurs, l'hydroxyéthylcellulose (HEC), le tensio-actif, le Butylène Glycol puis les pigments. Ajouter le copolymère acrylamide.
- Fondre la phase grasse à 95°C. Ajouter sur la phase aqueuse sous agitateur Rayneri, 10 minutes d'agitation puis laisser refroidir sous agitation lente. Vers 30°C, ajouter doucement le polyuréthane puis méthanol.

Produits utilisés dans cet exemple :
- le PEG 200 Stéarate de glycéryle vendu sous l'appellation SIMULSOL 220 par la société SEPIC S.A., Paris,
- le 1,3-Butylène glycol vendu par exemple par la société CELANESE CHEMICAL, Dallas,
- le Polysorbate 80 vendu sous l'appellation TWEEN 80 par la société UNIQUEMA,
- le Siméthicone vendu sous l'appellation MIRASIL SM par la société RHODIA SILICONES,
- le Polyuréthane dispersé dans l'eau est vendu sous l'appellation BAYCUSAN C 1001 par Bayer MaterialScience LLC, Pittsburgh, PA,

Les autres ingrédients étant aisément accessibles pour un homme du métier.

Les protocoles d'évaluation de tenue précédemment décrits ont donnés avec cette composition les résultats suivants :

| | | | |
|---|---|---|---|
| tenue à l'eau 1min | à l'eau 1h | au sébum 1h | à sec |
| 2 | 3 | 1 | 5 |

On observe ainsi en comparant les deux compositions que la composition selon l'invention renfermant un plastifiant lipophile du type Pemulen améliore considérablement la tenue à l'eau, qui est excellente.

La composition selon l'invention conserve en outre une excellente tenue au sébum et présente une bonne résistance aux frottements.

### Exemple 2 : Composition avec plastifiant lipophile

| **Ingrédients** | **%** |
|---|---|
| eau | 36,7 |
| Parabène | 0,25 |
| Phénoxyethanol et parabens | 1,2 |
| Hydroxybenzoate | 0,15 |
| EDTA | 0,2 |
| Dehydroacetate de sodium | 0,2 |
| PEG 200 glyceryl stéarate | 4 |
| 1, 3 Butylene glycol | 5 |
| Pigment noir | 7 |
| Copolymere acrylamide | 2,5 |
| Cire d'abeille | 7,4 |
| Cire de carnauba | 3,5 |
| Parabène | 0,05 |
| Simethicone | 0,1 |
| Dispersion de polyuréthane dans l'eau à 32% de matière active | 23,75 |
| Citrate de triéthyle | 5 |
| éthanol | 3 |

Cette formulation est préparée comme suit :
- Chauffer l'eau à 95°C et disperser sous Rayneri les conservateurs, le tensio-actif, le Butylène Glycol puis les pigments. Ajouter le copolymère acrylamide.
- Fondre la phase grasse à 95°C. Ajouter sur la phase aqueuse sous agitateur Rayneri, 10 minutes d'agitation puis laisser refroidir sous agitation lente. Vers 30°C, ajouter doucement le polyuréthane puis méthanol.

Produits utilisés dans cet exemple :
- le PEG 200 glyceryl stéarate vendu sous l'appellation SIMULSOL 220 par la société SEPIC S.A., Paris,
- le 1,3-Butylène glycol vendu par exemple par la société CELANESE CHEMICAL, Dallas,
- le Copolymère acrylamide vendu sous l'appellation SIMULGEL 600 par la société SEPPIC S.A., Paris,
- le Siméthicone vendu sous l'appellation MIRASIL SM par la société RHODIA SILICONES,
- le Polyuréthane dispersé dans l'eau est vendu sous l'appellation BAYCUSAN C 1001 par Bayer MaterialScience LLC, Pittsburgh, PA,
- le Citrate de triéthyle vendu sous l'appellation Citroflex® 2 par la société MORFLEX,

Les autres ingrédients étant aisément accessibles pour un homme du métier.

### Résultat

Les protocoles d'évaluation de tenue précédemment décrits ont donnés avec cette composition les résultats suivants :

| | | | |
|---|---|---|---|
| tenue à l'eau 1min | à l'eau 1h | au sébum 1h | à sec |
| 1 | 2 | 1 | 2 |

On observe ainsi en comparant les deux compositions que la composition selon l'invention renfermant un plastifiant lipophile du type Triethyl citrate améliore considérablement la tenue à l'eau, qui est excellente.

La composition selon l'invention conserve en outre une excellente tenue au sébum.

Cette composition présente par ailleurs une très bonne résistance aux frottements qui est significativement améliorée.

Dans toute la demande, le libellé « comprenant un » ou « comportant un » signifie « comprenant au moins un » ou « comportant au moins » un sauf si le contraire est spécifié.

## Revendications

1. Composition de maquillage des fibres kératiniques, telles que des cils ou des sourcils, aux propriétés démaquillantes améliorées, comprenant dans une phase aqueuse continue :
- une dispersion aqueuse de polyuréthane, le polyuréthane dispersé comprenant les produits de réaction de :
A) un prépolymère selon la formule : 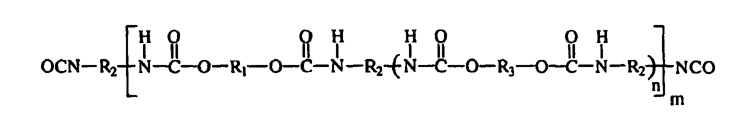 où
R₁ représente un radical hydrocarboné issu d'un polyesterpolyol, et notamment d'un polyesterdiol,
R₂ représente un radical hydrocarboné issu d'un polyisocyanate aliphatique ou cycloaliphatique,
R₃ représente un radical hydrocarboné issu d'un diol, éventuellement de bas poids moléculaire, éventuellement substitué par des groupes ioniques,
n vaut 0 à 5, et
m est > 1 ;
B) au moins un agent d'allongement de chaîne selon la formule :
H₂N―R₄―NH₂
où
R₄ représente un radical alkylène ou oxyde d'alkylène non substitué par des groupes ioniques ou potentiellement ioniques ; et
C) au moins un agent d'allongement de chaîne selon la formule :
H₂N-R₅-NH₂
où
R₅ représente un radical alkylène substitué par des groupes ioniques ou potentiellement ioniques ;
- ledit polyuréthane étant présent en une quantité en matière sèche supérieure ou égale à 5% en poids par rapport au poids total de ladite composition,
- ladite composition comprenant un système émulsionnant comprenant moins de 1 % en poids de triéthanolamine et comprenant une matière colorante choisie parmi les matières pulvérulentes.

2. Composition selon la revendication 1, dans laquelle ledit polyuréthane est présent en une quantité en matière sèche comprise inclusivement entre 5% et 15% en poids par rapport au poids total de ladite composition.

3. Composition selon la revendication 1 ou 2, dans laquelle ledit polyuréthane comprend des composés situés aux extrémités des chaînes et terminant lesdites chaînes, dérivant de composés présentant la formule : 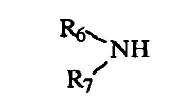 dans laquelle R₆ représente un atome d'hydrogène ou un radical alkylène présentant éventuellement une extrémité hydroxyle et R₇ représente un radical alkylène présentant éventuellement une extrémité hydroxyle.

4. Composition selon la revendication 1, 2 ou 3, dans laquelle le radical R1 est obtenu par (poly)condensation d'au moins un acide dicarboxylique avec au moins un diol, ledit acide dicarboxylique étant de préférence choisi parmi l'acide adipique, le diol étant de préférence choisi parmi l'hexanediol, le neopentylglycol, et leurs mélanges.

5. Composition selon l'une quelconque des revendications précédentes, dans laquelle le radical R2 est issu d'un polyisocyanate cycloaliphatique, et notamment un diisocyanate choisi parmi le 1,6-hexaméthylènedüsocyanate, l'isophoronediisocyanate et le dicyclohexylméthane diisocyanate, et leurs mélanges,

6. Composition selon l'une quelconque des revendications précédentes, dans laquelle le radical R3 est issu du néopentyl glycol.

7. Composition selon l'une quelconque des revendications précédentes, dans laquelle le radical R4 est choisi parmi l'éthylène diamine, la diéthanolamine er leurs mélanges.

8. Composition selon l'une quelconque des revendications précédentes, dans laquelle le radical R5 est choisi parmi les diaminosulfonates, et de préférence est le sel sodique de l'acide N-(2-aminoéthyl)-2-aminoéthanesulfonique.

9. Composition selon l'une quelconque des revendications précédentes, dans laquelle ladite composition de maquillage comprend un système émulsionnant choisi parmi :
i) un alkyl phosphate de métal alcalin de formule ou oxyde de phosphine (R-O)ₙ-P=O(O⁻M)ₘ avec R représentant un groupe alkyle, linéaire ou ramifié, en C₈-C₂₂ tel que le cétyle, n étant égal à 1, 2 ou 3 et m étant égal à 0, 1 ou 2, avec m + n étant égal à 3 et M représentant un atome d'hydrogène ou un métal alcalin ou alcalino terreux, préférentiellement n = 1 et m= 2, et M est un métal alcalin, tel que le sodium ou potassium ;
ii) un alcool polyéthoxylé de formule R'-(OCH₂CH₂)p-OH avec R' représentant un alkyle linéaire ou ramifié en C₁-C₃₀, , de préférence en C8-C24, mieux en en C₁₂-C₁₈ et particulièrement représente CH₃-(CH₂)₁₇- et p représentant un entier compris inclusivement entre 2 et 30, préférentiellement entre 2 et 20 ; tels que le steareth-20, le steareth-2 ;
iii) un sel d'acide glutamique de formule R-CONH-C(COO⁻M)-C₂H₄-COO-M' avec R représentant un groupe alkyle, linéaire ou ramifié en C₈-C₂₂ tel que le stéaryle et M' représentant un métal alcalin ou alcalino terreux ; et
iv) un alkyl glucoside obtenu par condensation de glucose et d'alcools gras linéaires ou ramifiés en C₈-C₂₂ tel qu'un mélange cetylique et stéarique nommé cétylstéaryle.

10. Composition selon l'une quelconque des revendications précédentes, dans laquelle le système émulsionnant comprend au moins un agent tensioactif choisi parmi le cétyl phosphate de potassium, le steareth 2, le steareth 20 et leurs mélanges.

11. Composition selon l'une quelconque des revendications précédentes, dans laquelle le système émulsionnant comprend au moins un agent tensioactif choisi parmi le glutamate de stéaroyle de sodium et le glucoside.de cétylstearyle, et leurs mélanges.

12. Composition selon l'une quelconque des revendications précédentes, comprenant en outre au moins un épaississant hydrophile choisi parmi les copolymères issus de la polymérisation :
(i) d'au moins un monomère de formule (1) suivante : 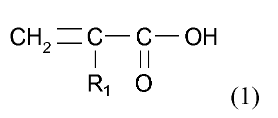 dans laquelle, R₁ désigne H ou CH₃ ou C₂H₅, c'est-à-dire des monomères acide acrylique, acide méthacrylique ou acide éthacrylique, et
(ii) d'au moins un monomère de type ester d'alkyl (C₁₀-C₃₀) d'acide carboxylique insaturé correspondant au monomère de formule (2) suivante : 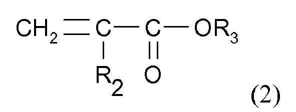 dans laquelle, R₂ désigne H ou CH₃ ou C₂H₅ et de préférence H ou CH₃, R₃ désignant un radical alkyle en C₁₀-C₃₀, et de préférence en C₁₂-C₂₂.

13. Composition selon la revendication 12, dans laquelle ledit épaississant hydrophile est choisi parmi des polymères issus de la polymérisation d'un mélange de monomères comprenant :
(i) essentiellement de l'acide acrylique,
(ii) un ester de formule (2) décrite ci-dessus et dans laquelle R₂ désigne H ou CH₃, R₃ désignant un radical alkyle ayant de 12 à 22 atomes de carbone, et
(iii) un agent réticulant, tel qu'un un monomère insaturé polyéthylénique copolymérisable, comme le phtalate de diallyle, le (méth)acrylate d'allyl, le divinylbenzène, le diméthacrylate de (poly)éthylèneglycol, et le méthylène-bis-acrylamide.

14. Composition selon l'une quelconque des revendications précédentes, comprenant en outre au moins un agent plastifiant lipophile présent à une quantité en poids supérieure ou égale à 2% par rapport au poids total de la composition.

15. Composition selon la revendication 14, dans laquelle ledit agent plastifiant est choisi parmi :
- les (poly)esters issus de la ou des réaction(s) d'au moins un acide carboxylique avec au moins un (poly)ol, tels que les esters d'acide citrique comme le citrate de triéthyle, le citrate de tributyle, l'acétylcitrate de triéthyle, l'acétylcitrate de tributyle, l'acétylcitrate de triéthyl-2 hexyle, et leurs mélanges,
- les éthers de glycol,
- le N-éthyl-o,p-toluènesulfonamide,
- les carbonates,
- les cétones,
et leurs mélanges.
